(19) Europäisches Patentamt
European Patent Office
Office européen des brevets

(11) EP 0 740 654 B1

(12) FASCICULE DE BREVET EUROPEEN

(45) Date de publication et mention de la délivrance du brevet: **13.10.1999 Bulletin 1999/41**

(21) Numéro de dépôt: **95907051.7**

(22) Date de dépôt: **19.01.1995**

(51) Int Cl.[6]: **C07C 281/04**, C07C 281/10, C07C 251/80, C07C 249/12, C07C 249/10, A61K 31/175, A61K 31/41, A61K 31/27, A61K 31/275

(86) Numéro de dépôt international: **PCT/FR95/00061**

(87) Numéro de publication internationale: **WO 95/19960 (27.07.1995 Gazette 1995/32)**

# (54) INHIBITEURS DE LA MONOAMINE OXYDASE B, LEURS PROCEDES DE PREPARATION ET LEURS APPLICATIONS

MONOAMIN OXYDASE B HEMMENDE VERBIDUNGEN VERFAHREN ZU IHRER HERSTELLUNG UN IHRER VERWENDUNG

MONOAMINE OXIDASE B INHIBITORS, PROCESSES FOR THEIR PREPARATION AND USE THEREOF

(84) Etats contractants désignés:
**AT BE CH DE DK ES FR GB GR IE IT LI LU MC NL PT SE**

(30) Priorité: **19.01.1994 FR 9400557**

(43) Date de publication de la demande: **06.11.1996 Bulletin 1996/45**

(73) Titulaires:
- **LABORATOIRES MAYOLY SPINDLER 78401 Chatou Cédex (FR)**
- **Seman, Michel F-75003 Paris (FR)**

(72) Inventeurs:
- **SEMAN, Michel F-75003 Paris (FR)**
- **BERNARD, Suzanne F-75007 Paris (FR)**
- **MILCENT René F-93600 Aulnay-sous-Bois (FR)**
- **PAILLAT, Catherine F-78400 Chatou (FR)**

(74) Mandataire: **Ores, Irène
CABINET ORES
6, Avenue de Messine
75008 Paris (FR)**

(56) Documents cités:

| | |
|---|---|
| EP-A- 0 254 461 | DE-B- 1 163 847 |
| GB-A- 839 610 | GB-A- 1 225 358 |
| US-A- 3 177 251 | US-A- 3 193 578 |

- **CHEMICAL ABSTRACTS, vol. 84, no. 21, 24 Mai 1976, Columbus, Ohio, US; abstract no. 150168j, GADZHIEV, G. 'Acylation of ethanolhydrazones' page 508 ;colonne 1 ; & AZERB. KHIM. ZH., no.5, 1975 pages 47 - 8**
- **CHEMICAL ABSTRACTS, vol. 76, no. 7, 14 Février 1972, Columbus, Ohio, US; abstract no. 33754w, STRUMILLO, J. ET. AL. 'Synthesis of hydrazino amines with potential antitubercular activity. VIII. Preparation of N-acetyl-N-(2-cyanoethyl)hydrazones of ketones.' page 280 ;colonne 1 ; & ACTA POL. PHARM., vol.28, no.3, 1971 pages 247 - 51**
- **CHEMICAL ABSTRACTS, vol. 68, no. 11, 11 Mars 1968, Columbus, Ohio, US; abstract no. 49206q, A. NOVACEK 'Acylation of some urea derivatives' page 4750 ;colonne 2 ; & COLLECT. CZECH. CHEM. COMMUN., vol.32, no.5, 1967 pages 1712 - 18**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 740 654 B1

- CHEMICAL ABSTRACTS, vol. 66, no. 19, 8 Mai 1967, Columbus, Ohio, US; abstract no. 85980e, A. NOVACEK ET. AL. 'Nucleic acid components and their analogs. LXXXVII. The preparation of 1-amino-5,6-dihydrouracil.' page 8063 ;colonne 2 ; & COLLECT. CZECH. CHEM. COMMUN., vol.32, no.1 pages 190 - 7
- JOURNAL OF THE CHEMICAL SOCIETY, PERKIN TRANSACTIONS 1, no.4, Avril 1987, LONDON, GB pages 885 - 97 G. LAWTON ET. AL. 'Regioselectivity in the Photochemical Ring Contraction of 4-Diazopyrazolidine-3,5-diones to give Aza-beta-lactams'
- CHEMICAL ABSTRACTS, vol. 76, no. 1, 3 Janvier 1972, Columbus, Ohio, US; abstract no. 3521a, A. NOVACEK ET. AL. 'Preparation and cyanoetylation of some hydrazine derivatives.' page 312 ;colonne 2 ; & COLLECT. CZECH. CHEM. COMMUN., vol.36, no.8 pages 3038 - 42

## Description

La présente Invention a pour objet des inhibiteurs de la Monoamine Oxydase B, leurs procédés de préparation et leurs applications en thérapeutique.

Les Monoamines Oxydases (MAO) sont des enzymes, localisées principalement dans la membrane externe de la mitochondrie et responsables de la métabolisation d'un certain nombre de monoamines jouant le rôle de neurotransmetteurs chez les mammifères.

Dans l'organisme humain, les principales monoamines qui sont désaminées par les MAO appartiennent soit à la série indolique : tryptamine et hydroxy-5 tryptamine ou sérotonine, soit à la série des acides aminés aromatiques : tyramine et catécholamines telles que dopamine, noradrénaline et adrénaline.

On distingue actuellement deux formes principales de MAO dans l'organisme : une forme A qui transformerait plus spécialement la sérotonine et la noradrénaline et une forme B dont les substrats préférentiels sont la benzylamine et la phényléthylamine, les deux formes transformant de façon à peu près équivalente la dopamine, la tyramine et la tryptamine.

Certains organes apparaissent n'exprimer qu'une des deux enzymes alors que les deux formes sont simultanément présentes dans d'autres tissus tels que le foie ou le cerveau.

Les hypothèses relatives aux mécanismes biochimiques de la dépression faisant intervenir certaines monoamines telles que la sérotonine et les catécholamines, il fut proposé, dès 1957, d'utiliser des inhibiteurs des MAO dans le traitement de cette pathologie.

Jusqu'à ce jour, se sont succédées trois générations d'inhibiteurs des MAO :

**1/ des inhibiteurs dits "mixtes" et "irréversibles" :**

Ils ont pour chefs de file la phénelzine (NARDELZINE®) et l'iproniazide (MARSILID®).

La structure commune à ces inhibiteurs est un groupement hydrazinique.

Ils sont dits "mixtes" car ils inhibent les deux formes de MAO. Ils sont dits "irréversibles" car ils forment une liaison covalente avec ces enzymes se traduisant par une inactivation irréversible de celles-ci qui ne prend fin que bien après l'arrêt du traitement, lorsque les enzymes nouvellement synthétisées ont pris le relais (15 à 21 jours). En outre, ils sont à l'origine d'une modification de la pression artérielle se traduisant par des troubles tensionnels indésirables : hypotension, vertiges, éblouissements, tendances hypothymiques et même syncopes.

**2/ des inhibiteurs "sélectifs" et "irréversibles" :**

Cette seconde génération d'IMAO regroupe trois familles de composés qui sont les arylhydrazines, les propargyliques et les cyclopropylamines.

Ces composés agissent préférentiellement sur l'une ou l'autre forme des MAO. Ainsi, par exemple, parmi les inhibiteurs propargyliques, la clorgyline et la pargyline inhibent la MAO A alors que le déprényl agit sur la MAO B.

Il convient toutefois de noter que, bien qu'inhibant les MAO, certains de ces composés tels que la pargyline et le déprényl sont dénués d'effets antidépresseurs et ont trouvé une indication dans le traitement de la maladie de Parkinson, en association avec la L-dopa.

En outre, tous ces composés agissent en se fixant d'abord aux MAO de façon non covalente, puis en formant avec elles des complexes covalents irréversibles, ce qui limite leur maniabilité en raison de la persistance de leur action bien après l'arrêt du traitement.

Ainsi, par exemple, une anesthésie générale ne peut être envisagée chez un patient traité par ce type d'IMAO qu'après une période d'environ trois semaines à l'issue de l'arrêt du traitement.

**3/ des inhibiteurs "sélectifs" et "réversibles" :**

Ces inhibiteurs de troisième génération regroupent :

- les dérivés de l'harmala, alcaloïdes de *Peganum harmala,* inhibiteurs sélectifs et réversibles de la MAO A, de durée d'action très courte ;
- les phénylalkylamines, inhibiteurs sélectifs de la MAO A ;
- les dérivés des oxazolidinones, inhibiteurs sélectifs de la MAO A et ayant pour chef de file la toloxatone (HUMORYL®). Ces composés sont décrits dans EP 0 424 243, EP 0 428 421 et EP 0 511 031.

Bien que l'utilisation de ces composés représente un progrès certain en thérapeutique en raison de leur activité rapidement réversible (en moins de 24 heures), certaines études physiopathologiques concernant les démences séniles et la maladie d'Alzheimer semblent montrer un intérêt tout particulier à l'utilisation des IMAO B.

En effet, dans ces pathologies, on observe une élévation importante du rapport MAO B / MAO A qui s'accompagne d'un accroissement d'une activité destructrice des MAO. La diminution du nombre de neurones produisant la dopamine en liaison avec le vieillissement cellulaire, contribue également à ce phénomène physiologique. Une éventuelle inhibition de la MAO B par des produits sélectifs et réversibles devrait permettre de rééquilibrer le rapport entre les deux formes d'enzyme en faveur de la MAO A, et d'améliorer ainsi l'état des sujets.

Les Demandes de Brevet EP 0 348 257 et WO 91/08201 proposent des dérivés d'arylméthyloxy-4 phényl diazole, essentiellement représentés par des tétrazoles, qui semblent présenter une activité anti-MAO sélective vis-à-vis de la MAO B. Cependant, on ne dispose pas, en ce qui concerne les composés décrits dans cette Demande, d'éléments sur la réversibilité de leur activité, ni d'un recul tant du point de vue pharmacologique que du point de vue toxicologique.

On connaît, par ailleurs, par le Brevet Allemand n° 1 163 847 des a-méthylbenzylhydrazones aptes à servir de produits intermédiaires dans la synthèse d'α-méthylbenzylhydrazides utiles comme anti-MAO. Toutefois, ces α-méthylbenzylhydrazones n'ont jamais été décrites comme présentant elles-mêmes des propriétés inhibitrices vis-à-vis de l'une ou de l'autre des formes de la MAO.

Les Demandeurs se sont en conséquence donné pour but de fournir de nouveaux inhibiteurs de la MAO B présentant à la fois une sélectivité et une réversibilité à l'égard de la MAO B, leur conférant une grande maniabilité thérapeutique.

La présente Invention a donc pour objet l'utilisation d'un composé répondant à la formule (I) suivante :

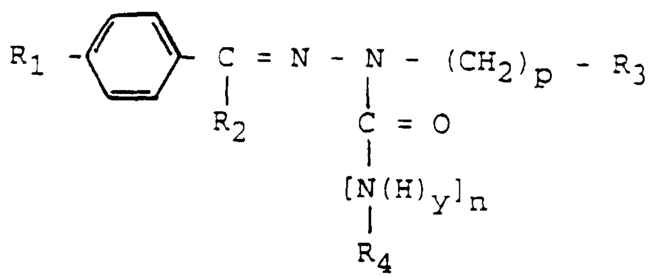

(I)

dans laquelle :

$R_1$ représente un atome d'hydrogène, un groupe phényléthoxy ou un groupe benzyloxy dont le noyau phényle est éventuellement substitué par un ou plusieurs atomes d'halogène et/ou un ou plusieurs groupes $NO_2$ et/ou un ou plusieurs groupes alkyle en $C_1$ à $C_3$ et/ou un ou plusieurs groupes alcoxy en $C_1$ à $C_3$ ;
p est un nombre entier de 0 à 3 ;
$R_3$ représente un atome d'hydrogène, un groupe CN, un groupe hydroxyle, un groupe CCH, un groupe alcoxy-carbonyle en $C_1$ à $C_3$ ou un groupe alkylé en $C_1$ à $C_3$ de cyanométhyle ;
$R_2$ représente un atome d'hydrogène ou un groupe alkyle en $C_1$ à $C_3$ ;
n est égal à 0 ou 1, auquel cas :

- si n = 0, $R_4$ représente un groupe alkyle en $C_1$ à $C_3$ ou un groupe alcoxy en $C_1$ à $C_3$; tandis que
- si n = 1, y = 1 et $R_4$ représente un groupe alkyle en $C_1$ à $C_3$, un groupe alcoxy en $C_1$ à $C_3$ ou un groupe phényle ; pour la préparation d'un médicament ayant une activité inhibitrice de la Monoamine Oxydase B.

Les composés de formule (I) tels que définis ci-dessus englobent leurs différents isomères.

Dans ce qui précède et ce qui suit, l'expression "alkyle en $C_1$ à $C_3$" désigne des groupes hydrocarbonés comprenant 1 à 3 atomes de carbone, à savoir méthyle, éthyle, n-propyle et i-propyle ; l'expression "alcoxy en $C_1$ à $C_3$" correspond à la formule O-alkyle en $C_1$ à $C_3$; l'expression "alcoxy-carbonyle en $C_1$ à $C_3$" représente une formule COO-alkyle en $C_1$ à $C_3$ ; le terme halogène désigne le chlore, le fluor, le brome ou l'iode.

Les composés de formule (I) tels que définis ci-dessus sont particulièrement utiles pour la préparation d'un médicament ayant une activité inhibitrice de la Monoamine Oxydase B car ils sont capables d'inhiber à la fois sélectivement et de manière réversible la MAO B tout en étant dépourvus d'effets toxiques.

Pour ce faire, ils peuvent être utilisés seuls ou en association, éventuellement avec un ou plusieurs autres principes actifs et/ou adjuvants pharmaceutiquement compatibles.

De façon préférée, $R_1$ représente un groupe benzyloxy dont le noyau phényle est éventuellement substitué par un ou plusieurs atomes d'halogène et/ou un ou plusieurs groupes $NO_2$ et/ou un ou plusieurs groupes alkyle en $C_1$

à $C_3$ et/ou un ou plusieurs groupes alcoxy en $C_1$ à $C_3$.

Parmi les composés de formule (I), certains ont déjà été décrits comme étant susceptibles de présenter des applications thérapeutiques. Toutefois, celles-ci concernent le traitement de la tuberculose (STRUMILLO et GRUDZINSKI, Acta Pol. Pharm., 1971, 28, 247-251) et le traitement d'affections virales rhinopharyngées (US 3,867,425) dans lesquels une inhibition de la Monoamine Oxydase B n'est en aucun cas recherchée.

L'Invention a donc également pour objet des médicaments nouveaux qui comprennent au moins un principe actif répondant à l'une des formules particulières suivantes :

(I-a)

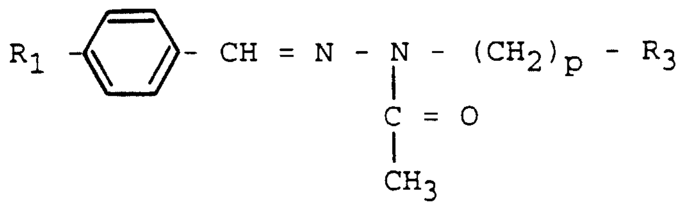

(I-b)

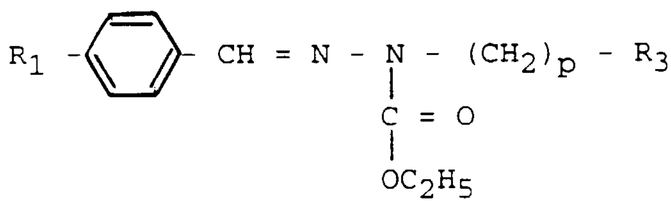

(I-c)

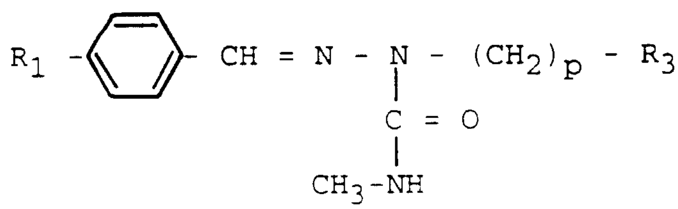

(I-d)

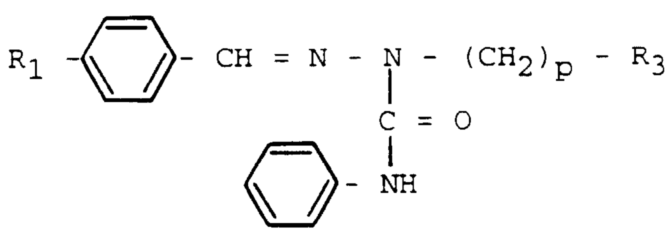

(I-e)

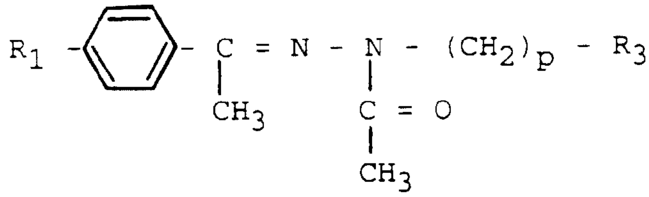

dans lesquelles :

$R_1$ représente un atome d'hydrogène, un groupe phényléthoxy ou un groupe benzyloxy dont le noyau phényle est éventuellement substitué par un ou plusieurs atomes d'halogène et/ou un ou plusieurs groupes $NO_2$ et/ou un ou plusieurs groupes alkyle en $C_1$ à $C_2$ et/ou un ou plusieurs groupes alcoxy en $C_1$ à $C_3$, à condition que $R_1$ soit différent d'un atome d'hydrogène dans la formule particulière (I-e) ;
p est un nombre entier de 0 à 3 ; et
$R_3$ représente un groupe CN, un groupe OH, un groupe CCH, un groupe alcoxy-carbonyle en $C_1$ à $C_3$ ou un groupe alkylé en $C_1$ à $C_3$ de cyanométhyle.

Ces médicaments trouvent notamment application dans le traitement des affections dans lesquelles une inhibition de la Monoamine Oxydase B telles que par exemple les syndromes dépressifs.
Parmi les médicaments conformes à l'Invention, on préfère notamment ceux comprenant comme principe(s) actif(s) les composés de formules particulières (I-a), (I-b), (I-c), (I-d) et (I-e) présentés dans le Tableau 1 ci-après :

**TABLEAU 1**

| N° de code | $R_1$ | p | $R_3$ |
|---|---|---|---|
| I-a1 | H | 2 | CN |
| I-a2 | benzyloxy | 2 | CN |
| I-a2.1 | (4-méthylbenzyl)oxy | 2 | CN |
| I-a2.2 | (4-nitrobenzyl)oxy | 2 | CN |
| I-a2.3 | (4-chlorobenzyl)oxy | 2 | CN |
| I-a2.4 | (4-méthoxybenzyl)oxy | 2 | CN |
| I-a2.5 | (2,4-dichlorobenzyl)oxy | 2 | CN |
| I-a2.6 | (2-chlorobenzyl)oxy | 2 | CN |
| I-a3 | H | 2 | OH |
| I-a4 | benzyloxy | 2 | OH |

**TABLEAU 1 (Suite)**

| | | | |
|---|---|---|---|
| I-a5 | benzyloxy | 2 | $COOCH_3$ |
| I-a6 | phényléthoxy | 2 | CN |
| I-a7 | benzyloxy | 1 | CH-CN<br>CH3 |
| I-a8 | benzyloxy | 1 | CN |
| I-a9 | benzyloxy | 3 | CN |
| I-a10 | benzyloxy | 1 | CCH |
| I-b1 | H | 2 | CN |
| I-b2 | benzyloxy | 2 | CN |
| I-b3 | H | 2 | OH |
| I-b4 | benzyloxy | 2 | OH |
| I-c1 | H | 2 | CN |
| I-c2 | benzyloxy | 2 | CN |
| I-c3 | H | 2 | OH |
| I-c4 | benzyloxy | 2 | OH |
| I-d1 | H | 2 | CN |
| I-d2 | benzyloxy | 2 | CN |
| I-e1 | benzyloxy | 2 | CN |

Dans un mode de réalisation préférée d'un médicament conforme à l'Invention, celui-ci comprend à titre de principe actif la 4-(benzyloxy)benzaldéhydeacétyl(2-cyanoéthyl)hydrazone (composé (I-a2).

Parmi les composés répondant aux formules particulières (I-a), (I-b), (I-c), (I-d) et (I-e), certains ont déjà été décrits, soit en tant qu'écrans solaires (US 3,419,659), soit en tant que produits résultant de l'acylation de l'urée (NOVACEK, Collect. Czech. Chem. Commun., 1967, 32, 1712-1718) ou encore en tant que pesticides (GADZHIEV et BUDAGOV, Azerb. Khim. Zh., 1975, 5, 47-48).

L'Invention a donc également pour objet des composés nouveaux qui répondent aux formules particulières suivantes :

(I-d)

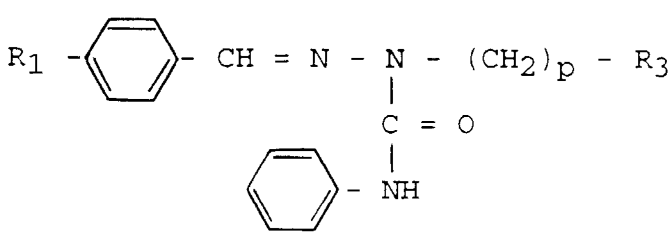

(I-e) 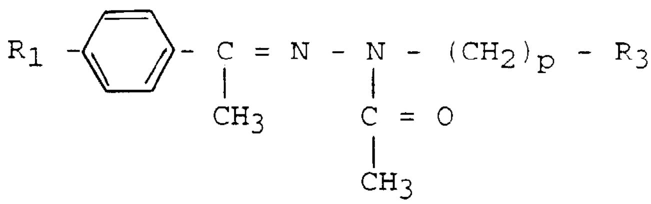

dans lesquelles :

$R_1$ représente un atome d'hydrogène, un groupe benzyloxy ou un groupe phényléthoxy, à condition que $R_1$ soit différent d'un atome d'hydrogène dans la formule particulière (I-e) ;
p est un nombre entier de 0 à 3 ; et
$R_3$ représente un groupe CN, un groupe OH, un groupe CCH, un groupe alcoxy-carbonyle en $C_1$ à $C_3$ ou un groupe alkylé en $C_1$ à $C_3$ de cyanométhyle.

La présente Invention a, par ailleurs, pour objet un procédé de préparation d'un composé de formule particulière (I-d) telle que définie ci-dessus, caractérisé en ce qu'il comprend :

1) une réaction de condensation entre un aldéhyde aromatique de formule :

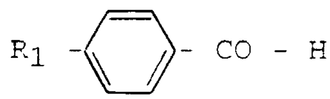

dans laquelle :
$R_1$ représente un atome d'hydrogène, un groupe benzyloxy ou phényléthoxy;
et une hydrazine de formule : $NH_2$ - NH - $(CH_2)_p$ - $R_3$ dans laquelle :

p est un nombre entier de 0 à 3 ;
$R_3$ représente un groupe CN, un groupe OH, un groupe CCH, un groupe alcoxy-carbonyle en $C_1$ à $C_3$ ou un groupe alkylé en $C_1$ à $C_3$ de cyanométhyle ;

2) une réaction d'acylation du produit issu de la réaction 1) au moyen d'un réactif tel que l'isocyanate de phényle.

Au demeurant, tous les composés de formule (I) peuvent être obtenus par ce procédé.
Ainsi, par exemple, pour la préparation des composés des formules particulières (I-a) et (I-b), l'acylation du produit issu de la réaction 1) (réaction de condensation entre l'aldéhyde aromatique et l'hydrazine) est réalisée au moyen d'un réactif de formule : $R_4$ - CO - Cl dans laquelle $R_4$ représente un groupe méthyle ou un groupe éthoxy tandis que pour la préparation des composés répondant à la formule particulière (I-c), cette même réaction d'acylation est réalisée au moyen de l'isocyanate de méthyle.
L'Invention a encore pour objet un procédé de préparation d'un composé de formule particulière (I-e), lequel procédé comprend :

1) une réaction de condensation entre un aldéhyde aromatique de formule :

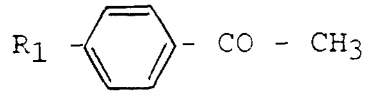

dans laquelle :

$R_1$ représente un groupe benzyloxy ou phényléthoxy ; et :

* soit une hydrazine de formule : $NH_2$ - NH - $(CH_2)_p$ - $R_3$ dans laquelle :

p est un nombre entier de 0 à 3 ;
$R_3$ représente un groupe CN, un groupe OH, un groupe CCH, un groupe alcoxy-carbonyle en $C_1$ à $C_3$ ou un groupe alkylé en $C_1$ à $C_3$ de cyanométhyle ;

* soit l'hydrazine de formule : $NH_2$ - NH - CO - $CH_3$ 2) une réaction :
* soit d'acylation du produit issu de la réaction 1) par un réactif tel que le chlorure d'acétyle ;
* soit d'alkylation du produit issu de la réaction 1) par un réactif de formule : Hal - $(CH_2)_p$ - C ≡ Z dans laquelle :

p est un nombre entier de 0 à 3 ;
Hal représente un atome d'halogène ;
Z représente un groupe CH ou un atome d'azote,
ou par un réactif de formule :
Br - $(CH_2)_p$ - COO - alkyle en $C_1$ à $C_3$ dans laquelle p est un nombre entier de 0 à 3.

De façon avantageuse, la réaction d'alkylation est réalisée avec un réactif choisi parmi le bromoacétonitrile, le 4-bromobutyronitrile, le bromure de propargyle ou le 3-bromopropionate de méthyle en présence d'un ou plusieurs solvants choisis parmi la pyridine anhydre, l'acétate d'éthyle anhydre, le benzène anhydre ou l'acide acétique.
L'Invention a enfin pour objet un procédé de traitement d'affections dans lesquelles est souhaitée une inhibition de la Monoamine Oxydase B, lequel procédé consiste à administrer à un hôte humain ou animal une quantité efficace d'au moins un composé de formule (I) telle que définie ci-avant.
Outre les dispositions qui précèdent, l'Invention comprend encore d'autres dispositions qui ressortiront de la description qui va suivre faite à titre d'exemples et en référence au dessin annexé dans lequel :

- la Figure 1 représente une gamme étalon pour déterminer la concentration en protéines d'une suspension mitochondriale ;
- la Figure 2 illustre l'activité inhibitrice vis-à-vis de la MAO A et de la MAO B du composé (I-a2) ;
- la Figure 3 illustre l'activité inhibitrice vis-à-vis de la MAO A et de la MAO B du composé (I-a4) ;
- la Figure 4 illustre sous forme d'histogrammes la reversibilité de l'activité inhibitrice vis-à-vis de la MAO B du composé (I-a2).

Il doit être bien entendu, toutefois, que ces exemples sont donnés uniquement à titre d'illustrations de l'objet de l'Invention dont ils ne constituent en aucune manière une limitation.

**EXEMPLES :**

**Exemple 1 : préparation du composé (I-a1) : benzaldéhyde-acétyl(2-cyanoéthyl)hydrazone**

**1.1 Préparation de la (2-cyanoéthyl)hydrazone du benzaldéhyde :**

La (2-cyanoéthyl)hydrazone du benzaldéhyde est préparée par condensation du benzaldéhyde avec la (2-cyanoéthyl)hydrazine selon le protocole suivant : à une solution contenant $10^{-2}$ moles de benzaldéhyde dans 20 ml d'éthanol, on ajoute $10^{-2}$ moles de (2-cyanoéthyl) hydrazine. La réaction est laissée à reflux pendant 30 minutes. En fin de réaction, le mélange est refroidi. Après évaporation des solvants, le produit obtenu se présente sous forme d'une huile extrêmement instable. Le rendement brut de la réaction est de 81%.

La (2-cyanoéthyl)hydrazine peut être obtenue par une réaction de MICHAEL (HOFFMAN V. et JACOBI B., Chemical Abstracts, Vol. 28, p 5473, 1934), c'est à dire par action de l'hydrazine en excès sur une mole d'acrylonitrile dans l'éthanol.

**1.2 Acylation de la (2-cyanoéthyl)hydrazone du benzaldéhyde par le chlorure d'acétyle :**

A une solution contenant $10^{-2}$ moles de (2-cyanoéthyl)hydrazone du benzaldéhyde et 0,79 g ($10^{-2}$ moles) de pyridine anhydre dans 20 ml d'acétate d'éthyle anhydre, sous agitation à froid, on ajoute, goutte à goutte, 0,78 g ($10^{-2}$ moles) de chlorure d'acétyle.

Le mélange est porté lentement à reflux et laissé sous agitation deux heures, puis filtré à chaud. Après refroidissement du filtrat, le produit cristallise. Il est filtré. Une quantité supplémentaire de celui-ci est obtenue par évaporation du filtrat. Le produit obtenu en fin de réaction est lavé avec 10 ml d'eau, puis séché et recristallisé dans l'éthanol. Le produit a un point de fusion égal à 109°C. Le rendement de la réaction est de 54%.

**Exemple 2 : Préparation du composé (I-a2) : 4-(benzyloxy) benzaldéhyde-acétyl (2-cyanoéthyl)hydrazone**

**2.1 Préparation de la (2-cyanoéthyl)hydrazone du 4-(benzyloxy)benzaldéhyde :**

La (2-cyanoéthyl)hydrazone du 4-(benzyloxy) benzaldéhyde est préparée par condensation du 4-(benzyloxy) benzaldéhyde avec la (2-cyanoéthyl)hydrazine selon le protocole suivant :

à une solution contenant $10^{-2}$ moles de 4-(benzyloxy) benzaldéhyde dans 20 ml d'éthanol, on ajoute $10^{-2}$ moles de (2-cyanoéthyl)hydrazine. La réaction est laissée à reflux pendant 30 minutes. En fin de réaction, le produit précipite. Après filtration, il est récupéré et recristallisé deux fois dans l'éthanol. Le rendement de la réaction est de 86%.

**2.2 Acylation de la (2-cyanoéthyl)hydrazone du 4-(benzyloxy)benzaldéhyde par le chlorure d'acétyle :**

Cette réaction d'acylation est effectuée selon un protocole identique à celui décrit au point 1.2 de l'exemple 1 ci-dessus, à partir d'une part, d'une solution contenant $10^{-2}$ moles de (2-cyanoéthyl)hydrazone du 4-(benzyloxy) benzaldéhyde et 0,79 g ($10^{-2}$ moles) de pyridine anhydre dans 20 ml d'acétate d'éthyle anhydre et d'autre part, de 0,78 g ($10^{-2}$ moles) de chlorure d'acétyle. Elle permet d'obtenir le composé (I-a2) avec un rendement de 50%. Il présente un point de fusion de 150°C.

**Exemple 3 : Préparation du composé (I-a3) : benzaldéhyde-acétyl(2-hydroxyéthyl)hydrazone**

**3.1 Préparation de la (2-hydroxyéthyl)hydrazone du benzaldéhyde :**

La (2-hydroxyéthyl)hydrazone du benzaldéhyde est préparée par condensation du benzaldéhyde avec la (2-hydroxyéthyl)hydrazine selon le protocole suivant : à une solution contenant $10^{-2}$ moles de benzaldéhyde dans 20 ml d'éthanol, on ajoute $10^{-2}$ moles de (2-hydroxyéthyl)hydrazine. La réaction est laissée à reflux pendant 30 minutes. Puis, le mélange est refroidi. Les solvants sont éliminés par évaporation. Le produit obtenu est une huile extrêmement instable. Le rendement brut de la réaction est de 97%.

La (2-hydroxyéthyl)hydrazine est un produit commercialisé par ALDRICH.

**3.2 Acylation de la (2-hydroxyéthyl)hydrazone du benzaldéhyde par le chlorure d'acétyle :**

A une solution contenant $10^{-2}$ moles de (2-hydroxyéthyl)hydrazone du benzaldéhyde et 0,79 g ($10^{-2}$ moles) de pyridine anhydre dans 20 ml d'acétate d'éthyle anhydre, sous agitation à froid, on ajoute, goutte à goutte, 0,78 g ($10^{-2}$ moles) de chlorure d'acétyle.

Le mélange réactionnel est laissé à 0°C sous agitation pendant deux heures, puis il est filtré. Le produit obtenu en fin de réaction est lavé avec 10 ml d'eau, puis séché et recristallisé dans l'éthanol. Le rendement de la réaction est de 34%. Le produit a un point de fusion de 110°C.

**Exemple 4 : Préparation du composé (I-a4) : 4-(benzyloxy) benzaldéhyde-acétyl(2-hydroxyéthyl)hydrazone**

**4.1 Préparation de la (2-hydroxyéthyl)hydrazone du (4-benzyloxy)benzaldéhyde :**

La (2-hydroxyéthyl)hydrazone du 4-(benzyloxy)-benzaldéhyde est obtenue par condensation du 4-(benzyloxy) benzaldéhyde avec la (2-hydroxyéthyl) hydrazine, en faisant réagir par exemple une solution contenant $10^{-2}$ moles de (4-benzyloxy)benzaldéhyde dans 20 ml d'éthanol avec $10^{-2}$ moles de (2-hydroxyéthyl) hydrazine. La réaction est laissée à reflux pendant 30 minutes, à l'issue desquelles le mélange est refroidi et l'hydrazone obtenue précipite. Après filtration, elle est récupérée et recristallisée deux fois dans l'éthanol. Elle présente un point de fusion de 91°C. Le rendement de la réaction est de 79%.

**4.2 Acylation de la (2-hydroxyéthyl)hydrazone du 4-(benzyloxy)benzaldéhyde par le chlorure d'acétyle :**

Elle est effectuée selon un protocole identique à celui décrit au point 3.2 de l'exemple 3 ci-avant, à partir d'une part, d'une solution contenant $10^{-2}$ moles de (2-hydroxyéthyl)hydrazone du 4-(benzyloxy) benzaldéhyde et 0,79 g ($10^{-2}$ moles) de pyridine anhydre dans 200 ml d'acétate d'éthyle anhydre et d'autre part, 0,78 g ($10^{-2}$ moles) de chlorure d'acétyle.

Elle permet d'obtenir le composé (I-a4) avec un rendement de 38%. Il présente un point de fusion de 131°C.

**Exemple 5 : Préparation du composé (I-a2. 6) : 4-[(2-chlorobenzyl)oxy]benzaldéhyde-acétyl(2-cyanoéthyl) hydrazone**

Le composé (I-a2.6) est obtenu par une réaction d'alkylation *in situ* réalisée selon le protocole suivant : à une solution de $5.10^{-3}$ moles de 4-[(2-chlorobenzyl) oxy]benzaldéhyde dans 20 ml de benzène anhydre, sont ajoutés 0,43 g ($5,1.10^{-3}$ moles) de 2-(cyanoéthyl) hydrazine, à température ambiante et sous agitation. Le mélange réactionnel est porté à reflux et l'eau formée lors de la réaction est éliminée par distillation azéotropique. La solution est alors ramenée à 10°C et successivement 0,40 g ($5,1.10^{-3}$ moles) de pyridine anhydre, puis 0,39 g ($5,1.10^{-3}$ moles) de chlorure d'acétyle sont ajoutés. Le mélange est laissé sous agitation à température ambiante pendant 30 minutes. Le benzène est alors éliminé par évaporation sous vide. Le résidu obtenu est dissout dans 100 ml de dichlorométhane et la phase organique résultante est lavée trois fois à l'eau. Elle est ensuite séchée sur du sulfate de magnésium, puis évaporée. Le résidu est recristallisé dans l'éthanol. Il présente un point de fusion de 142°C. Le rendement de la réaction est de 51%.

**Exemple 6 : Préparation du compose (I-a2.5) : 4-[(2,4-dichlorobenzyl)oxy]benzaldéhyde-acétyl(2-cyanoéthyl) hydrazone**

Le composé (I-a2.5) est préparé selon un protocole identique à celui décrit dans l'exemple 5, à partir de 4-[(2,4-dichlorobenzyl)oxy]benzaldéhyde et de 2-(cyanoéthyl)hydrazine.

Le composé (I-a2.5) a un point de fusion de 137°C. Le rendement de la réaction est de 38%.

**Exemple 7 : Préparation du composé (I-a6) : 4-(2-phényléthoxy)benzaldéhyde-acétyl(2-cyanoéthyl)hydrazone**

Le composé (I-a6) est préparé selon un protocole identique à celui décrit dans l'exemple 5, à partir de 4-(2-phényléthoxy)benzaldéhyde et de 2-(cyanoéthyl)hydrazine. Il présente un point de fusion à 129°C. Le rendement de la réaction est de 55%.

**Exemple 8 : Préparation du composé (I-a7) : 4-(benzyloxy)benzaldéhyde-acétyl(2-cyanopropyl)hydrazone**

A une solution de $5.10^{-3}$ moles de 4-(benzyloxy)benzaldéhyde dans 20 ml de benzène anhydre, sont ajoutés 0,64 g ($5,1.10^{-3}$ moles) de 2-(cyanopropyl) hydrazine, à température ambiante et sous agitation. Le mélange réactionnel est porté à reflux et l'eau formée lors de la réaction est éliminée par distillation azéotropique. La solution est alors ramenée à 10°C et successivement 0,40 g ($5,1.10^{-3}$ moles) de pyridine anhydre, puis 0,39 g ($5,1.10^{-3}$ moles) de chlorure d'acétyle sont ajoutés. Le mélange est laissé sous agitation à température ambiante pendant 30 minutes. Le benzène est alors éliminé par évaporation sous vide. Le résidu obtenu est dissout dans 100 ml de dichlorométhane et la phase organique résultante est lavée trois fois à l'eau. Elle est ensuite séchée sur du sulfate de magnésium, puis évaporée. Le résidu est recristallisé dans l'éthanol. Il présente un point de fusion de 106°C. Le rendement de la réaction est de 42%.

**Exemple 9 : Préparation du composé (I-a5) : 4-(benzyloxy)benzaldéhyde-acétyl (2-méthoxycarbonyléthyl) hydrazone**

**9.1 Préparation de la 4-(benzyloxy)benzaldéhyde-acétylhydrazone :**

A $10^{-2}$ moles de 4-(benzyloxy)benzaldéhyde (2,12 g) dans 20 ml d'éthanol, est ajoutée une solution de $10^{-2}$ moles d'acétylhydrazine (0,74 g) dans 20 ml d'éthanol. Le mélange réactionnel est porté à reflux pendant 90 minutes à l'issue desquelles la solution est lentement ramenée à 0°C. Le produit obtenu précipite. Il est filtré, essoré et recristallisé dans l'éthanol. Point de fusion : 170°C. Rendement de la réaction = 76%.

**9.2 Alkylation de la 4-(benzyloxy)benzaldéhyde-acétylhydrazone :**

A $5.10^{-3}$ moles d'hydrure de sodium (0,12 g) en suspension dans 10 ml de DMF anhydre, on ajoute une solution de $5.10^{-3}$ moles de 4-(benzyloxy)benzaldéhyde-acétylhydrazone (1,34 g) dans 5 ml de DMF anhydre. Le mélange réactionnel est chauffé lentement au bain-marie à 80°C pendant 30 minutes. Après refroidissement, une solution contenant 0,85 g ($5,1.10^{-3}$ moles) de 3-bromopropionate de méthyle dans 5 ml de DMF est ajoutée lentement au mélange. Celui-ci est agité pendant une heure à température ambiante, puis il est versé dans 100 ml d'eau glacée. Un produit précipite. Après 30 minutes de repos, il est filtré, soumis à un essorage à l'issue duquel il est chromatographié sur gel de silice 60 MACHEREY-NAGEL (0,04-0,063 ml) en utilisant un mélange d'acétate d'éthyle et de dichlorométhane (1/9) comme éluant. L'huile obtenue précipite. Le composé (I-a5) est recristallisé lentement

dans l'éther. Son point de fusion est de 170°C. Le rendement de la réaction est de 11%.

**Exemple 10 : Préparation du composé (I-a8) : 4-(benzyloxy)benzaldéhyde-acétyl(cyanométhyl)hydrazone**

Le composé (I-a8) est obtenu par une réaction d'alkylation de la 4-(benzyloxy)benzaldéhydeacétylhydrazone, préparée conformément au point 9.1 de l'exemple 9 ci-dessus, par le bromoacétonitrile.

L'alkylation par le bromoacétonitrile est réalisée selon le protocole suivant : à $5.10^{-3}$ moles d'hydrure de sodium (0,12 g) en suspension dans 10 ml de DMF anhydre, on ajoute une solution de $5.10^{-3}$ moles de 4-(benzyloxy) benzaldéhyde-acétylhydrazone (1,34 g) dans 5 ml de DMF anhydre. Le mélange réactionnel est chauffé lentement au bain-marie à 80°C pendant 30 minutes. Après refroidissement, une solution contenant 0,61 g (5, $1.10^{-3}$ moles) de 3-bromoacétonitrile dans 5 ml de DMF est ajoutée lentement au mélange. Celui-ci est agité pendant une heure à température ambiante, puis il est versé dans 100 ml d'eau glacée. Un produit précipite. Après 30 minutes de repos, il est filtré, essoré puis recristallisé dans l'éthanol. Le composé (I-a8) a un point de fusion est de 136°C. Le rendement de la réaction est de 59%.

**Exemple 11 : Préparation du composé (I-a9) : 4-(benzyloxy)benzaldéhydo-acétyl(3-cyanopropyl)hydrazone**

Le composé I-a9 est obtenu par une réaction d'alkylation de la 4-(benzyloxy)benzaldéhyde-acétylhydrazone, préparé comme décrit au point 9.1 de l'exemple 9 ci-avant, par du 4-bromobutyronitrile.

La réaction d'alkylation est réalisée selon un protocole identique à celui décrit dans l'exemple 10 ci-dessus, à l'aide d'une solution contenant 0,75 g (5,1. $10^{-3}$ moles) de 4-bromobutyronitrile dans 5 ml de DMF. Elle présente un rendement de 75%. Le composé (I-a9) obtenu a un point de fusion de 103°C.

**Exemple 12 : Préparation du composé I-(a10) : 4-(benzyloxy)benzaldéhyde-acétylpropargylhydrazone**

Ce composé est obtenu par une réaction d'alkylation de la 4-(benzyloxy)benzaldéhyde-acétylhydrazone, préparée comme décrit au point 9.1 de l'exemple 9, par du bromure de propargyle.

La réaction d'alkylation est réalisée selon un protocole identique à celui décrit dans l'exemple 10 ci-avant, à l'aide d'une solution contenant 0,60 g (5,$1.10^{-3}$ moles) de bromure de propargyle dans 5 ml de DMF, avec un rendement de 54%. Le composé (I-a10) a un point de fusion de 120°C.

**Exemple 13 : Préparation du composé (I-b1) : benzaldéhyde-(2-cyanoéthyl)(éthoxycarbonyl)hydrazone**

**13.1 Préparation de la (2-cyanoéthyl)hydrazone du benzaldéhyde :**

La (2-cyanoéthyl)hydrazone du benzaldéhyde est préparée en utilisant un protocole identique à celui décrit au point 1.1 de l'exemple 1.

**13.2 Acylation de la (2-cyanoéthyl)hydrazone du benzaldéhyde par le chloroformiate d'éthyle :**

A une solution contenant $10^{-2}$ moles de (2-cyanoéthyl)hydrazone du benzaldéhyde et 0,79 g ($10^{-2}$ moles) de pyridine anhydre dans 20 ml d'acétate d'éthyle anhydre, sous agitation à froid, on ajoute, goutte à goutte, 1,08 g ($10^{-2}$ moles) de chloroformiate d'éthyle en solution dans 5 ml d'acétate d'éthyle anhydre. Le mélange réactionnel est porté lentement à reflux et laissé sous agitation 2 heures, puis filtré à chaud.

Après refroidissement du filtrat, le produit cristallise. Il est filtré. Une quantité supplémentaire de produit peut être obtenue par évaporation du filtrat. Le produit est lavé avec 10 ml d'eau, puis séché et recristallisé dans un mélange d'acétate d'éthyle et d'éther de pétrole. Il présente un point de fusion de 91°C. Le rendement de la réaction est de 48%.

**Exemple 14 : Préparation du composé (I-b2) : 4-(benzyloxy)benzaldéhyde-(2-cyanoéthyl)(éthoxycarbonyl) hydrazone**

**14.1 Préparation de la (2-cyanoéthyl)hydrazone du 4-(benzyloxy)benzaldéhyde :**

La (2-cyanoéthyl)hydrazone du 4-(benzyloxy) benzaldéhyde est obtenue selon un procédé identique à celui décrit au point 2.1 de l'exemple 2 ci-avant.

**14.2 Acylation de la (2-cyanoéthyl)hydrazone du 4-(benzyloxy)benzaldéhyde par le chloroformiate d'éthyle :**

Elle est effectuée selon un protocole identique à celui décrit au point 13.2 de l'exemple 13 ci-dessus, à partir d'une part, d'une solution contenant $10^{-2}$ moles de (2-cyanoéthyl)hydrazone du 4-(benzyloxy) benzaldéhyde et 0,79 g ($10^{-2}$ moles) de pyridine anhydre dans 20 ml d'acétate d'éthyle anhydre et d'autre part, d'une solution contenant 1,08 g ($10^{-2}$ moles) de chloroformiate d'éthyle dans 5 ml d'acétate d'éthyle anhydre.

Le produit obtenu est recristallisé dans l'éthanol. Le rendement de la réaction est de 71%. Le produit a un point de fusion égal à 100°C.

**Exemple 15 : Préparation du composé (I-b3) : benzaldéhyde-(2-hydroxyéthyl)(éthoxycarbonyl)hydrazone**

Ce composé est obtenu par une réaction d'acylation de la (2-hydroxyéthyl)hydrazone du benzaldéhyde, préparée conformément au point 3.1 de l'exemple 3, par du chloroformiate d'éthyle.

Cette acylation est réalisée de la façon suivante :

à une solution contenant $10^{-2}$ moles de (2-hydroxyéthyl) hydrazone du benzaldéhyde et 0,79 g ($10^{-2}$ moles) de pyridine anhydre dans 20 ml d'acétate d'éthyle anhydre, sous agitation à froid, on ajoute, goutte à goutte, 1,08 g ($10^{-2}$ moles) de chloroformiate d'éthyle dans 5 ml d'acétate d'éthyle anhydre. Le mélange réactionnel est laissé à 0°C sous agitation durant 2 heures, puis il est filtré. Le produit obtenu est lavé avec 10 ml d'eau, puis séché et recristallisé dans un mélange d'acétate d'éthyle et d'éther de pétrole. Il présente un point de fusion de 69,5°C. Le rendement de la réaction est de 62%.

**Exemple 16 : Préparation du composé (I-b4) : 4-(benzyloxy)benzaldéhyde-(2-hydroxyéthyl)(éthoxycarbonyl) hydrazone**

Ce composé est obtenu par une réaction d'acylation de la (2-hydroxyéthyl)hydrazone du 4-(benzyloxy)benzaldéhyde, préparée conformément au point 4.1 de l'exemple 4 ci-avant, par du chloroformiate d'éthyle.

La réaction d'acylation est effectuée selon un protocole identique à celui décrit dans l'exemple 15 ci-dessus, à l'exception de la recristallisation du produit qui est obtenue dans l'éthanol. Le rendement de cette réaction d'acylation est de 71%. Le produit a un point de fusion égal à 118°C.

**Exemple 17 : Préparation du composé (I-c1) : benzaldéhyde-(2-cyanoéthyl)(N-méthylcarbamoyl)hydrazone**

**17.1 Préparation de la (2-cyanoéthyl)hydrazone du benzaldéhyde :**

La (2-cyanoéthyl)hydrazone du benzaldéhyde est obtenue selon le protocole décrit au point 1.1 de l'exemple 1 ci-avant.

**17.2 Acylation de la (2-cyanoéthyl)hydrazone du benzaldéhyde par l'isocyanate de méthyle :**

A une solution de $10^{-2}$ moles de (2-cyanoéthyl) hydrazone du benzaldéhyde dans 30 ml de benzène anhydre, sont ajoutés, à 25°C, 0,57 g ($10^{-2}$ moles) d'isocyanate de méthyle. Le mélange est ensuite porté à ébullition et laissé sous reflux pendant 30 minutes. Par refroidissement, le composé acylé cristallise. Il est filtré, essoré, puis recristallisé dans un mélange de benzène et de cyclohexane. Son point de fusion est de 145°C. Le rendement de la réaction est de 48%.

**Exemple 18 : Préparation des composés (I-c2), (I-c3) et (I-c4):**

Les composés (I-c2), (I-c3) et (I-c4) sont obtenus par une réaction d'acylation respectivement de la (2-cyanoéthyl)hydrazone du 4-(benzyloxy)benzaldéhyde, de la (2-hydroxyéthyl)hydrazone du benzaldéhyde et de la (2-hydroxyéthyl)hydrazone du 4-(benzyloxy)benzaldéhyde, par l'isocyanate de méthyle.

Les (2-cyanoéthyl)hydrazones et les (2-hydroxyéthyl)hydrazones d'aldéhydes aromatiques sont préparées conformément aux points 2.1 de l'exemple 2, 3.1 de l'exemple 3 et 4.1 de l'exemple 4.

La réaction d'acylation de ces hydrazones d'aldéhydes aromatiques par l'isocyanate de méthyle est effectuée selon un protocole identique à celui décrit au point 17.2 de l'exemple 17 ci-dessus, exception faite de la recristallisation du composé (I-c4) qui est obtenue dans l'éthanol.

Le rendement de cette acylation est respectivement :

- de 41% pour le composé (I-c2) [4-(benzyloxy) benzaldéhyde-(2-cyanoéthyl)(N-méthylcarbamoyl)hydrazone], dont le point de fusion est de 152°C,
- de 35% pour le composé (I-c3) [benzaldéhyde-(2-hydroxyéthyl) (N-méthylcarbamoyl)hydrazone], dont le point de fusion est de 141,5°C,

- de 42% pour le composé (I-c4) [4-(benzyloxy) benzaldéhyde-(2-hydroxyéthyl)(N-méthylcarbamoyl) hydrazone], dont le point de fusion est de 196°C.

**Exemple 19 : Préparation des composés (I-d1) et (I-d2)** :

Les composés (I-dl) et (I-d2) sont préparés par une réaction d'acylation *in situ* réalisée selon le protocole suivant : à une solution de 1 g ($10^{-2}$ moles) de benzaldéhyde (pour la préparation du composé (I-d1)) ou de 4-(benzyloxy)-benzaldéhyde (pour la préparation du composé (I-d2)) dans 30 ml- de benzène anhydre, on ajoute 0,8 g ($10^{-2}$ moles) de (2-cyanoéthyl)hydrazine. Le mélange réactionnel est porté à ébullition jusqu'à disparition de l'aldéhyde dont la présence est détectée par une chromatographie sur couche mince. L'eau est alors enlevée par distillation azéotropique. La solution est ensuite refroidie à 25°C. On ajoute alors 1,3 g ($1,1.10^{-2}$ moles) d'isocyanate de phényle. Le mélange est porté à ébullition pendant 5 minutes. Un produit blanc se forme. Il est filtré, puis lavé avec 5 ml de benzène. La recristallisation du produit est réalisée dans du butanol.

Le rendement de la réaction est :

- quantitatif pour le composé (I-d1) [benzaldéhyde-(2-cyanoéthyl)(N-phénylcarbamoyl)hydrazone], dont le point de fusion est de 125°C,
- égal à 51% pour le composé (I-d2) [4-(benzyloxy) benzaldéhyde-(2-cyanoéthyl)(N-phénylcarbamoyl)hydrazone], dont le point de fusion est de 194°C.

**Exemple 20 : Préparation du composé (I-e1) : 4-(benzyloxy)acétophénone-acétyl(2-cyanoéthyl)hydrazone**

A une solution contenant 0,99 g ($4,4.10^{-3}$ moles) de 4-(benzyloxy)acétophénone dans 20 ml de benzène anhydre, en présence de quelques gouttes d'acide acétique, on ajoute $4,4.10^{-3}$ moles (0,37 g) de 2-(cyanoéthyl)hydrazine, à température ambiante et sous agitation. Le mélange réactionnel est porté à reflux et l'eau formée lors de la réaction est éliminée par distillation azéotropique. La solution est alors ramenée à 10°C et successivement $4,4.10^{-3}$ moles de pyridine anhydre (0,34 g) et $4,4.10^{-3}$ moles de chlorure d'acétyle (0,34 g) sont ajoutées. Le mélange est laissé sous agitation à température ambiante pendant 30 minutes. Le benzène est alors éliminé par évaporation sous vide. Le résidu obtenu est dissout dans 100 ml de dichlorométhane et la phase organique résultante est lavée trois fois à l'eau. Elle est ensuite séchée sur du sulfate de magnésium, puis évaporée. Le résidu est recristallisé dans l'éthanol. Il présente un point de fusion de 77°C. Le rendement de la réaction est de 15%.

**Exemple 21 : Etudes pharmacologiques** :

**21.1 Activité et sélectivité**

Les composés de formule (I) ont été testés in vitro afin de déterminer leur activité inhibitrice vis-à-vis de la MAO A et de la MAO B du cerveau de rat et leur sélectivité à l'égard de ces enzymes.

**A/ Protocole expérimental :**

a) Préparation de la suspension mitochondriale :

* Prélèvement des tissus cérébraux

Des rats Whistar pesant entre 150 et 200 grammes sont tués par décapitation. Le cerveau, à l'exception du cervelet est prélevé, pesé et homogénéisé à l'Ultraturax (à vitesse maximum pendant 5 secondes et ce, cinq fois de suite) dans un tampon saccharose 0,32 M, Tris 10 mM, de pH 7,4 à 0°C, à raison de 10 ml de tampon par gramme de tissu frais.

* Préparation de la suspension mitochondriale

L'homogénat précèdemment obtenu est centrifugé pendant 5 minutes à 1000g dans une centrifugeuse réfrigérée (+4°C). Le surnageant est récupéré et soumis à une nouvelle centrifugation dans les mêmes conditions afin de parfaire l'élimination des débris vasculaires, des noyaux et des gros fragments myéliniques. Une dernière centrifugation du surnageant à 20.000g pendant 20 minutes permet d'obtenir un culot enrichi en mitochondries.

Le culot est repris dans du tampon phosphate (mono et bipotassique) 100 mM de pH 7,4, à raison de 4 ml de tampon par gramme de tissu frais.

La préparation enzymatique ainsi obtenue est fractionnée dans des tubes à raison de 0,5 ml de solution par tube et conservée à -80°C. Une conservation d'une durée de quelques mois n'entraîne aucune perte

d'activité.

b) Dosage de l'activité Monoamine Oxydase (MAO)

* Principe de la mesure de l'activité enzymatique MAO *in vitro*

La sérotonine (5HT) et la β-phényléthylamine (β-PEA) qui constituent les substrats spécifiques respectivement des MAO A et B, sont utilisées pour mesurer chaque activité. Ces molécules sont marquées sur leur chaîne latérale par du carbone 14 et sont transformées par les MAO, par désamination oxydative, respectivement en hydroxy-5 indolacétaldéhyde et en β-phénylacétaldéhyde selon le schéma ci-dessous :

*

$$\text{5-HT} + O_2 + H_2O \xrightarrow{\text{MAO}} \text{hydroxy-5 indolacétaldéhyde} + H_2O_2 + NH_3$$

*

$$\beta\text{-PEA} + O_2 + H_2O \xrightarrow{\text{MAO}} \beta\text{-phénylacétaldéhyde} + H_2O_2 + NH_3$$

Les mitochondries sont incubées en présence d'un des deux substrats radioactifs. Après un temps déterminé, la réaction enzymatique est arrêtée par précipitation des protéines mitochondriales. Les produits radioactifs sont ensuite extraits par un mélange de solvants organiques. L'activité MAO est alors déterminée par comptage de la radioactivité des produits de la réaction.

* Méthode de dosage L'essai standard est réalisé avec :

- 90 µl de tampon phosphate mono et bipotassique 100 mM, de pH 7,4,
- 50 ml de suspension mitochondriale,
- 20 µl de mélange $H_2O$/DMSO correspondant à la dilution du produit à tester dans le cas du témoin ou 20 µl d'une solution d'inhibiteur à tester à la concentration choisie,
- 40 µl de substrat radioactif de radioactivité égale à 0,05 µCI,

pour un volume final de 200 µl.

La réaction nécessaire au dosage est réalisée soit à l'issue d'une préincubation de l'enzyme (MAO A ou MAO B) avec l'inhibiteur à tester d'une durée de 30 minutes dans un bain thermostaté à 37°C, soit sans préincubation. Dans tous les cas, la réaction est déclenchée par l'addition de 40 µl d'une solution $10^{-5}$ molaire de 5-HT marquée au carbone 14 (de concentration radioactive = 1,25 µCI/ml) ou d'une solution $5.10^{-6}$ molaire de β-PEA marquée au carbone 14 (de concentration radioactive = 1,25 µCI/ml).

L'incubation du mélange réactionnel est maintenue pendant 60 minutes pour la 5-HT et 10 minutes pour la β-PEA, à l'issue desquelles la réaction est arrêtée par précipitation des protéines en ajoutant 200 ml d'HCL 4N froid, et en agitant immédiatement le mélange obtenu à l'aide d'un vortex pendant 5 secondes.

L'extraction des produits de la réaction est réalisée par l'ajout d'1 ml d'un mélange de toluène et d'acétate d'éthyle (1/1) suivi d'une agitation au vortex pendant 30 secondes et d'une centrifugation à 3000 tours/minute pendant 5 minutes. 500 µl de surnageant sont prélevés et comptés dans 2 ml de toluène contenant du diphényloxazole à 0,4% au moyen d'un RackBéta 1290 dont le rendement est de 94%.

Le blanc de la réaction consiste à opérer de la même manière que pour l'essai standard mais le substrat est ajouté après précipitation des protéines mitochondriales.

* Conditions cinétiques du dosage

Les réactions enzymatiques sont étudiées dans les conditions suivantes :

- la concentration de l'enzyme est très inférieure à celle des substrats,
- seule la phase initiale de la réaction est suivie.

Ces deux conditions réunies fixent le cadre d'une méthode cinétique enzymatique à l'état stationnaire

de la réaction.

L'activité résiduelle de l'enzyme est déterminée par la formule suivante :

$$\frac{\text{(cpm en présence de l'inhibiteur - cpm du blanc)}}{\text{(cpm du témoin - cpm du blanc)}} \times 100$$

* Détermination de la concentration en protéines de la suspension mitochondriale

Elle est réalisée à l'aide du kit de dosage Protein Assay Reagent de la Société PIERCE CHEMICAL Co.

La densité optique, lue à la longueur d'onde de 550 nm est proportionnelle à la concentration protéique d'un échantillon biologique.

La concentration en protéines de la suspension mitochondriale est déterminée par comparaison avec une gamme étalon de sérum albumine bovine diluée dans le tampon de suspension des mitochondries.

La Figure 1 présente cette gamme étalon, avec en ordonnée la densité optique mesurée à 550 nm (DO 550 nm) et, en abcisse la concentration en sérum albumine bovine (SAB) exprimée en mg/ml.

La suspension mitochondriale présente une densité optique égale à 0,238 à une dilution au 1/15ème, et égale à 0,124 à une dilution au 1/30ème. La concentration en protéines est donc de 6 mg/ml. Elle est ramenée par dilution à une concentration de travail de 0,6 mg/ml.

**B/ Résultats** :

Les $CI_{50}$ des inhibiteurs testés vis-à-vis de la MAO A et de la MAO B, qui représentent les concentrations d'inhibiteurs capables d'obtenir une inhibition de 50% de ces enzymes, sont déterminées graphiquement à partir des courbes de pourcentage d'activité résiduelle des MAO pour différentes concentrations d'inhibiteurs.

Les Figures 2 et 3 montrent, à titre d'exemples, les courbes de pourcentage d'activité résiduelle de la MAO A et de la MAO B obtenues respectivement avec les composés (I-a2) et (I-a4), d'une part sans préincubation de l'enzyme et du composé (sp), d'autre part après une préincubation de l'enzyme et de l'inhibiteur (ap) . Sont exprimés, en ordonnées, le pourcentage d'activité enzymatique résiduelle et, en abcisses, le logarithme en base 10 de la concentration en inhibiteur.

Le Tableau 2 présente les valeurs des $CI_{50}$, exprimées en moles/litre, vis-à-vis de la MAO A et vis-à-vis de la MAO B, de différents composés de formule (I), obtenues d'une part sans préincubation de l'enzyme et du composé et, d'autre part avec une préincubation de l'enzyme et de l'inhibiteur.

TABLEAU 2

| N° de code | | $CI_{50}$ MAO A (M) | $CI_{50}$ MAO B (M) |
|---|---|---|---|
| I-a2 | sp | nd | $3,5.10^{-8}$ |
| | ap | nd | $3.10^{-9}$ |
| I-a2.1 | sp | nd | $4,3.10^{-5}$ |
| | ap | nd | $6,9.10^{-7}$ |
| I-a2.2 | sp | nd | $3.10^{-6}$ |
| | ap | nd | $1,4.10^{-6}$ |
| I-a2.3 | sp | nd | $1,13.10^{-5}$ |
| 22 | ap | nd | $2,45.10^{-7}$ |
| I-a2.4 | sp | nd | $2,8.10^{-5}$ |
| | ap | nd | $2,15.10^{-6}$ |
| I-a2.5 | sp | nd | précipité |
| | ap | nd | $3,8.10^{-7}$ |
| I-a4 | sp | $5.10^{-5}$ | $2,8.10^{-8}$ |
| | ap | $5.10^{-5}$ | $6,3.10^{-9}$ |
| I-a6 | sp | $5,3.10^{-5}$ | $3.10^{-6}$ |
| | ap | $6,8.10^{-6}$ | $1,5.10^{-8}$ |
| I-a10 | sp | nd | $4,1.10^{-6}$ |
| | ap | nd | $3,6.10^{-7}$ |

TABLEAU 2 (suite)

| N° de code | | $CI_{50}$ MAO A (M) | $CI_{50}$ MAO B (M) |
|---|---|---|---|
| I-b2 | sp | nd | $2{,}2.10^{-5}$ |
| | ap | nd | $6{,}3.10^{-6}$ |
| I-b4 | sp | nd | $3{,}5.10^{-5}$ |
| | ap | nd | $10^{-5}$ |
| I-c1 | sp | nd | $2{,}8.10^{-4}$ |
| | ap | nd | $1{,}2.10^{-4}$ |
| I-c2 | sp | nd | $1{,}8.10^{-6}$ |
| | ap | nd | $2{,}5.10^{-6}$ |
| sp : sans préincubation<br>ap : avec préincubation<br>nd : non déterminable, inhibition trop faible | | | |

**21.2 Réversibilité de l'activité inhibitrice à l'égard des MAO B**

La réversibilité de l'activité inhibitrice des composés de formule (I) est déterminée en comparant les pourcentages d'activité résiduelle de la Monoamine Oxydase B, obtenus avant et après lavage d'un mélange d'enzyme et d'inhibiteur ayant été préalablement soumis à une incubation d'une durée de 30 minutes à 37°C.

**A/ Principe :**

L'enzyme en solution dans du tampon phosphate et l'inhibiteur sont mis à incuber pendant 30 minutes à 37°C. A l'issue de cette incubation, une partie de la solution est soumise à un test d'activité comme décrit ci-après, ce qui permet de déterminer les pourcentages d'activité enzymatique avant lavage de la solution enzyme + inhibiteur. Le reste de la solution est dilué 10 fois avec du tampon phosphate, puis centrifugé pendant 20 minutes à 27000 g dans une centrifugeuse réfrigérée. Les surnageants sont enlevés. Les culots sont repris dans un volume de tampon phosphate correspondant à la quantité de surnageant enlevé. Une centrifugation est réalisée dans les mêmes conditions que précédemment. Les surnageants sont éliminés. Les culots sont à nouveau repris dans un volume de tampon phosphate égal au volume de surnageant éliminé. Une centrifugation est à nouveau réalisée et les culots sont repris avec la quantité minimale de tampon phosphate nécessaire au test d'activité, qui permet de définir les pourcentages d'activité enzymatique après lavage de la solution enzyme + inhibiteur.

Le déprényl, inhibiteur irréversible de la MAO B, sert de produit de référence.

Un témoin est réalisé en utilisant du DMSO (diméthylsulfoxyde) à 1% à la place de l'inhibiteur.

**B/ Test d'activité :**

Chaque test est réalisé avec :

- 160 µl de solution contenant la MAO B et l'inhibiteur ou le DMSO dans du tampon phosphate,
- 40 µl de solution 5 µM de β-phényléthylamine marquée par du carbone 14 (radioactivité = 0,25 µCi).

La réaction est déclenchée par l'ajout des 40 µl de β-phényléthylamine. Le temps de réaction est d'une minute. La réaction est arrêtée par ajout de 200 µl d'HCl 4N puis on ajoute 1 ml d'un mélange d'acétate d'éthyle et de toluène (1/1) pour extraire le produit radioactif. Les tubes sont mis à centrifuger pendant 5 minutes à 3000 tours par minute dans une centrifugeuse réfrigérée. 2 ml de scintillant sont ajoutés à 500 µl de surnageant directement dans les tubes. Le comptage est réalisé sur 120 secondes.

**C/ Résultats :**

Le Tableau 3 présente les pourcentages moyens d'activité MAO résiduelle obtenus à partir de 4 essais, pour le composé (I-a2) et le déprényl, avant et après lavage du mélange enzyme + inhibiteur.

TABLEAU 3

| Inhibiteur | Pourcentage d'activité MAO résiduelle | |
|---|---|---|
| | Avant lavage | Après lavage |
| Composé (I-a2) | 14 | 105 |
| Déprényl | 25,6% | 14,7% |

La Figure 4 montre sous forme de trois histogrammes les résultats du tableau 3 ainsi que la perte d'activité enzymatique liée au temps. Est exprimé, en ordonnées, le pourcentage d'activité enzymatique résiduelle.

Le premier histogramme correspond aux pourcentages moyens d'activité MAO résiduelle obtenus respectivement avec le témoin (T), le déprényl (D) et le composé (I-a2) avant lavage du mélange. Le second histogramme correspond aux pourcentages moyens d'activité MAO résiduelle obtenus respectivement avec le témoin (T), le déprényl (D) et le composé (I-a2) après lavage du mélange. Le troisième histogramme montre, pour le témoin et les inhibiteurs testés, la perte d'activité enzymatique liée au temps.

**21.3 Toxicité**

L'évaluation de la cytotoxicité des composés de formule (I) a fait l'objet de deux tests différents :

- un test MTT (3-[4,5-diméthylthiazol-2-yl]-2,5-diphényltétrazolium bromide) sur les cellules adhérentes,
- un test sur les cellules en suspension.

**A/ Test MTT sur les cellules adhérentes :**

On utilise des cellules épithéliales Hela Ohio. Cette lignée cellulaire est issue d'un cancer de col de l'utérus humain. Les cellules sont cultivées dans un milieu EMEM supplémenté avec 10% de sérum de veau foetal.

Les composés conformes à l'Invention sont testés à des concentrations comprises entre $10^{-4}$ et $10^{-8}$ molaire dans du DMSO à 75%.

Dans une plaque de 96 puits, $3.10^4$ cellules Hela dans 100 µl de milieu de culture sont déposées par puits. Les plaques sont incubées pendant 18 heures, à une température de 37°C, en atmosphère humide avec 5% de $CO_2$. Les cellules peuvent ainsi adhérer à leur support. Dans les puits, on ajoute une solution de composé à tester de concentration comprise entre $10^{-4}$ et $10^{-8}$ molaire ou une solution de DMSO à 75% destinée à obtenir des témoins de croissance.

Après une incubation d'une durée de 24 heures, en atmosphère humide, avec 5% de $CO_2$, les cellules sont lavées par du PBS. Puis, 10 µl d'une solution contenant 5 mg/ml de MTT dans du PBS sont ajoutés avec 100 µl de milieu dans chaque puit. Les plaques sont incubées pendant 4 heures à 37°C en atmosphère humide et avec 5% de $CO_2$. Le MTT est métabolisé dans les cellules vivantes en un composé violet insoluble. A l'issue de l'incubation, les puits sont lavés à l'aide de PBS pour éliminer le MTT en suspension. 100 µl de DMSO ajoutés aux puits provoquent la lyse des cellules et la solubilisation du produit coloré. Les plaques sont agitées pendant 15 minutes pour homogénéiser le milieu et la suspension de produit violet. La concentration du métabolite dans les puits est lue par photométrie à une longueur d'onde de 550 nm et le nombre de cellules vivantes est fonction de la densité optique obtenue. L'index de viabilité est calculé ainsi :

$$\text{Index de viabilité} = 1 - \frac{DO_t - DO}{DO_t}$$

$DO_t$ étant la densité optique obtenue pour des cellules traitées par le DMSO et DO étant la densité optique pour des cellules traitées par un composé conforme à l'Invention.

**B/ Test réalisé sur les cellules en suspension :**

On utilise des cellules Daudi. Cette lignée cellulaire est issue d'un lymphome de Burkit humain. Les cellules sont cultivées dans un milieu RPMI supplémenté avec 10% de sérum de veau foetal.

Les composés conformes à l'Invention sont utilisés à des concentrations comprises entre $10^{-4}$ et $10^{-8}$ molaire dans du DMSO à 75%.

Chaque puit d'une plaque de 24 puits est ensemencé par 100 µl d'une suspension cellulaire contenant $2.10^5$ cellules par ml. Chaque puit reçoit un composé à tester, à une concentration comprise entre $10^{-4}$ et $10^{-8}$ molaire ou du DMSO à 75% (témoins de croissance).

Les plaques sont incubées pendant 24 heures à 37°C, en atmosphère humide et avec 5% de $CO_2$. Après homogénéisation du milieu, 100 ml de suspension sont prélevés du puit et mélangés à 100 ml de bleu Trypan à 0,04% dans le PBS. Le bleu Trypan diffuse dans les cellules mortes mais pas dans les cellules vivantes.

Les cellules vivantes (réfringeantes) et mortes (bleues) sont énumérées sur cellule de Malassez. La fraction cellulaire vivante (Fv) est égale au rapport entre le nombre de cellules vivantes et le nombre total de cellules. L'index de viabilité est égal à :

$$Fv = \frac{\text{cellules vivantes}}{\text{cellules vivantes et mortes}}$$

$$\text{Index de viabilite} = 1 - \frac{Fvt - Fv}{Fvt}$$

Fvt étant la fraction vivante des cellules traitées par le DMSO et Fv étant la fraction vivante des cellules traitées par les composés conformes à l'Invention.

**C/ Résultats :**

Le Tableau 4 ci-après présente les résultats des tests de cytotoxicité réalisés avec des composés de formule (I). Deux valeurs ont été retenues pour chaque type de tests :

* la dose léthale 50 (DL 50) correspondant à la concentration, exprimée en moles/litre, de composé pour laquelle 50% des cellules sont tuées ;

* la concentration maximale de composé (CM 100), exprimée en moles/litre, pour laquelle toutes les cellules sont vivantes.

TABLEAU 4

| N° de code | Test MTT sur cellules adhérentes | | Test sur cellules en suspension | |
|---|---|---|---|---|
| | DL 50 (M) | CN 100 (M) | DL 50 (M) | CN 100 (M) |
| I-a2 | $10^{-4}$ | $10^{-5}$ | $10^{-4}$ | $10^{-5}$ |
| I-a2.2 | $3.10^{-5}$ | $6,5.10^{-7}$ | $2,2.10^{-5}$ | $10^{-5}$ |
| I-a2.3 | $2,2.10^{-5}$ | $6.10^{-7}$ | $5.10^{-5}$ | $10^{-5}$ |
| I-a2.4 | $4.10^{-5}$ | $2,3.10^{-6}$ | $6,5.10^{-5}$ | $1,25.10^{-5}$ |
| I-a4 | $10^{-4}$ | $10^{-5}$ | $10^{-4}$ | $10^{-5}$ |

## Revendications

1. Utilisation d'un composé de formule (I) :

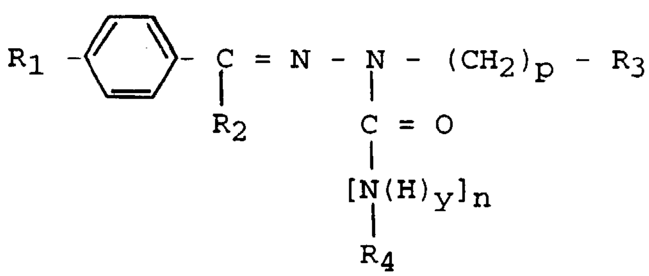

(I)

dans laquelle :

$R_1$ représente un atome d'hydrogène, un groupe phényléthoxy ou un groupe benzyloxy dont le noyau phényle est éventuellement substitué par un ou plusieurs atomes d'halogène et/ou plusieurs groupes $NO_2$ et/ou un ou plusieurs groupes alkyle en $C_1$ à $C_3$ et/ou un ou plusieurs groupes alcoxy en $C_1$ à $C_3$ ;
p est un nombre entier de 0 à 3 ;
$R_3$ représente un atome d'hydrogène, un groupe CN, un groupe hydroxyle, un groupe CCH, un groupe alcoxy-carbonyle en $C_1$ à $C_3$ ou un groupe alkylé en $C_1$ à $C_3$ de cyanométhyle ;
$R_2$ représente un atome d'hydrogène ou un groupe alkyle en $C_1$ à $C_3$ ;
n est égal à 0 ou 1, auquel cas

- si n = 0, $R_4$ représente un groupe alkyle en $C_1$ à $C_3$ ou un groupe alcoxy en $C_1$ à $C_3$, tandis que
- si n = 1, y = 1 et $R_4$ représente un groupe alkyle en $C_1$ à $C_3$, un groupe alcoxy en $C_1$ à $C_3$ ou un groupe phényle ; pour la préparation d'un médicament ayant une activité inhibitrice de la Monoamine Oxydase B.

2. Utilisation d'un composé selon la Revendication 1, caractérisée en ce que $R_1$ représente un groupe benzyloxy

dont le noyau phényle est éventuellement substitué par un ou plusieurs atomes d'halogène et/ou un ou plusieurs groupes $NO_2$ et/ou un ou plusieurs groupes alkyles en $C_1$ à $C_3$ et/ou un ou plusieurs groupes alcoxy en $C_1$ à $C_3$.

**3.** Utilisation d'un composé selon la Revendication 1 ou la Revendication 2, caractérisée en ce que le composé est choisi parmi les composés suivants :

- 4-(benzyloxy)benzaldéhyde-acétyl(2-cyanoéthyl)hydrazone (I-a2),
- 4-[(4-méthylbenzyl)oxy]benzaldéhyde-acétyl(2-cyanoéthyl)hydrazone (I-a2.1),
- 4-[(4-nitrobenzyl)oxy]benzaldéhyde-acétyl(2-cyanoéthyl)hydrazone (I-a2.2),
- 4-[(4-chlorobenzyl)oxy]benzaldéhyde-acétyl(2-cyanoéthyl)hydrazone (I-a2.3),
- 4-[(4-méthoxybenzyl)oxy]benzaldéhyde-acétyl(2-cyanoéthyl)hydrazone (I-a2.4),
- 4-[(2,4-dichlorobenzyl)oxy]benzaldéhyde-acétyl(2-cyanoéthyl)hydrazone (I-a2.5),
- 4-[(2-chlorobenzyl)oxy]benzaldéhyde-acétyl(2-cyanoéthyl)hydrazone (I-a2.6),
- 4-(benzyloxy)benzaldéhyde-acétyl(2-hydroxyéthyl)hydrazone (I-a4),
- 4-(benzyloxy)benzaldéhyde-acétyl(2-méthoxycarbonyléthyl)hydrazone (I-a5),
- 4-(2-phényléthoxy)benzaldéhyde-acétyl(2-cyanoéthyl)hydrazone (I-a6),
- 4-(benzyloxy)benzaldéhyde-acétyl(2-cyanopropyl)hydrazone (I-a7),
- 4-(benzyloxy)benzaldéhyde-acétyl(cyanométhyl)hydrazone (I-a8),
- 4-(benzyloxy)benzaldéhyde-acétyl(3-cyanopropyl)hydrazone (I-a9),
- 4-(benzyloxy)benzaldéhyde-acétylpropargylhydrazone (1-a10),
- 4-(benzyloxy)benzaldéhyde-(2-cyanoéthyl)(éthoxycarbonyl)hydrazone (I-b2),
- 4-(benzyloxy)benzaldéhyde-(2-hydroxyéthyl)(éthoxycarbonyl)hydrazone (I-b4),
- benzaldéhyde-(2-cyanoéthyl)(N-méthylcarbamoyl)hydrazone (I-c1),
- 4-(benzyloxy)benzaldéhyde-(2-cyanoéthyl)(N-méthylcarbamoyl)hydrazone) (I-c2),
- benzaldéhyde-(2-cyanoéthyl)(N-phénylcarbamoyl)hydrazone (I-d1),
- 4-(benzyloxy)benzaldéhyde-(2-cyanoéthyl)(N-phénylcarbamoyl)hydrazone (I-d2), et
- 4-(benzyloxy)acétophénone-acétyl(2-cyanoéthyl) hydrazone (I-el).

**4.** Médicament, caractérisé en ce qu'il comprend au moins un principe actif qui répond à l'une des formules particulières suivantes :

(I-a) 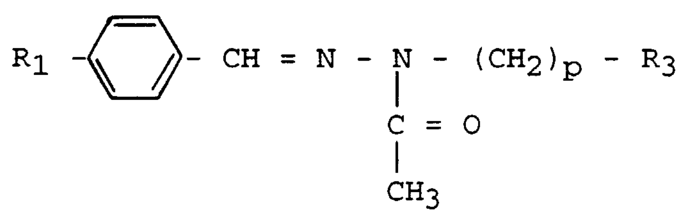

(I-b) 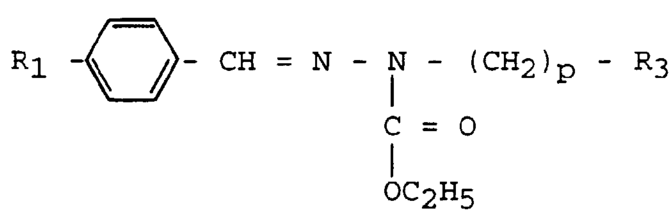

(I-c)

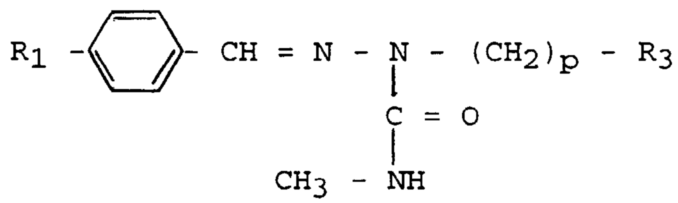

(I-d)

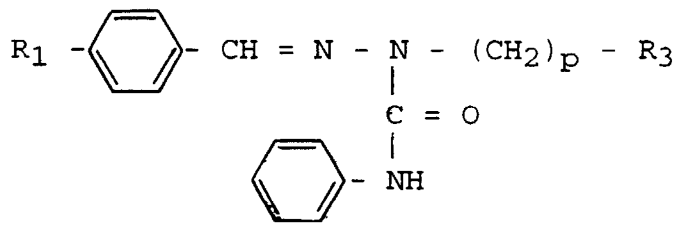

(I-e)

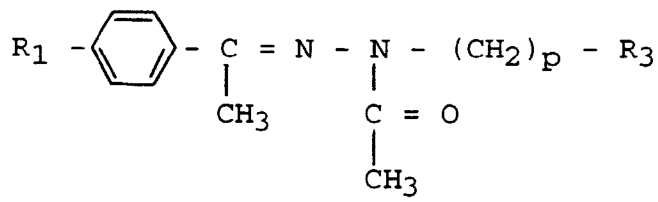

dans lesquelles :

$R_1$ représente un atome d'hydrogène, un groupe phényléthoxy ou un groupe benzyloxy dont le noyau phényle est éventuellement substitué par un ou plusieurs atomes d'halogène et/ou un ou plusieurs groupes $NO_2$ et/ou un ou plusieurs groupes alkyle en $C_1$ à $C_3$ et/ou un ou plusieurs groupes alcoxy en $C_1$ à $C_3$, à condition que $R_1$ soit différent d'un atome d'hydrogène dans la formule particulière (I-e) ;
p est un nombre entier de 0 à 3 ;
$R_3$ représente un groupe CN, un groupe OH, un groupe CCH, un groupe alcoxycarbonyle en $C_1$ à $C_3$ ou un groupe alkylé en $C_1$ à $C_3$ de cyanométhyle.

**5.** Médicament selon la Revendication 4, caractérisé en ce que $R_1$ représente un groupe benzyloxy dont le noyau phényle est éventuellement substitué par un ou plusieurs atomes d'halogène et/ou un ou plusieurs groupes $NO_2$ et/ou un ou plusieurs groupes alkyle en $C_1$ à $C_3$ et/ou un ou plusieurs groupes alcoxy en $C_1$ à $C_3$.

**6.** Médicament selon la Revendication 4, caractérisé en ce que le principe actif est choisi parmi les composés suivants :

. 4-(benzyloxy)benzaldéhyde-acétyl(2-cyanoéthyl)hydrazone (I-a2),
. 4-[(4-méthylbenzyl)oxy]benzaldéhyde-acétyl(2-cyanoéthyl)-hydrazone (I-a2.1),
. 4-[(4-nitrobenzyl)oxy]benzaldéhyde-acétyl(2-cyanoéthyl)-hydrazone (I-a2.2),
. 4-[(4-chlorobenzyl)oxy]benzaldéhyde-acétyl(2-cyanoéthyl)- hydrazone (I-a2.3),
. 4-[(4-méthoxybenzyl)oxy]benzaldéhyde-acétyl(2-cyanoéthyl)hydrazone (I-a2.4),
. 4-[(2,4-dichlorobenzyl)oxy]benzaldéhyde-acétyl(2-cyanoéthyl)hydrazone (I-a2.5),
. 4-[(2-chlorobenzyl)oxy]benzaldéhyde-acétyl(2-cyanoéthyl)hydrazone (I-a2.6),
. 4-(benzyloxy)benzaldéhyde-acétyl(2-hydroxyéthyl)hydrazone (I-a4),
. 4-(benzyloxy)benzaldéhyde-acétyl(2-méthoxycarbonyléthyl) hydrazone (I-a5),
. 4-(2-phényléthoxy)benzaldéhyde-acétyl(2-cyanoéthyl)hydrazone (I-a6),
. 4-(benzyloxy)benzaldéhyde-acétyl(2-cyanopropyl)hydrazone (I-a7),
. 4-(benzyloxy)benzaldéhyde-acétyl(cyanométhyl)hydrazone (I-a8),

. 4-(benzyloxy)benzaldéhyde-acétyl(3-cyanopropyl)hydrazone (I-a9),
. 4-(benzyloxy)benzaldéhyde-acétylpropargylhydrazone (1-a10),
. 4-(benzyloxy)benzaldéhyde-(2-cyanoéthyl)(éthoxycarbonyl)hydrazone (I-b2),
. 4-(benzyloxy)benzaldéhyde-(2-hydroxyéthyl)(éthoxycarbonyl)hydrazone (I-b4),
. benzaldéhyde-(2-cyanoéthyl)(N-méthylcarbamoyl)hydrazone (I-c1),
. 4-(benzyloxy)benzaldéhyde-(2-cyanoéthyl) (N-méthylcarbamoyl)hydrazone) (I-c2),
. benzaldéhyde-(2-cyanoéthyl)(N-phénylcarbamoyl)hydrazone (I-d1),
. 4-(benzyloxy)benzaldéhyde-(2-cyanoéthyl)(N-phénylcarbamoyl)hydrazone (I-d2), et
. 4-(benzyloxy)acétophénone-acétyl(2-cyanoéthyl)hydrazone (I-e1).

**7.** Médicament selon la Revendication 6, caractérisé en ce que le principe actif est la 4-(benzyloxy)benzaldéhyde-acétyl(2-cyanoéthyl)hydrazone (I-a2).

**8.** Composé caractérisé en ce qu'il répond à l'une des formules particulières :

(I-d)

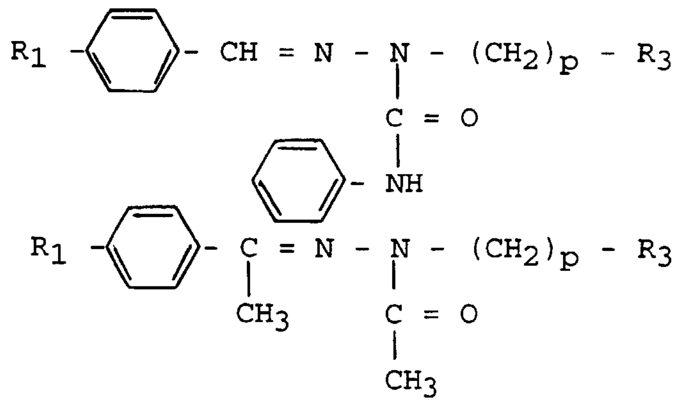

(I-e)

dans lesquelles :

$R_1$ représente un atome d'hydrogène, un groupe benzyloxy ou un groupe phényléthoxy, à condition que $R_1$ soit différent d'un atome d'hydrogène dans la formule particulière (I-e) ;
p est un nombre entier de 0 à 3 ;
$R_3$ représente un groupe CN, un groupe OH, un groupe CCH, un groupe alcoxycarbonyle en $C_1$ à $C_3$ ou un groupe alkylé en $C_1$ à $C_3$ de cyanométhyle.

**9.** Composé selon la Revendication 8, caractérisé en ce qu'il est choisi parmi les composés suivants :

. benzaldéhyde-(2-cyanoéthyl)(N-phénylcarbamoyl)hydrazone (I-d1),
. 4-(benzyloxy)benzaldéhyde-(2-cyanoéthyl)(N-phénylcarbamoyl)hydrazone (I-d2), et
. 4-(benzyloxy)acétophénone-acétyl(2-cyanoéthyl) hydrazone (I-e1).

**10.** Procédé de préparation d'un composé de formule particulière (I-d) selon la Revendication 8 ou la Revendication 9, caractérisé en ce qu'il comprend :

1) une réaction de condensation entre un aldéhyde aromatique de formule :

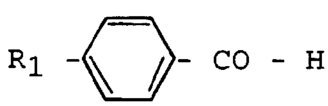

dans laquelle :
$R_1$ représente un atome d'hydrogène, un groupe benzyloxy ou phényléthoxy ;
et une hydrazine de formule : $NH_2$- NH - $(CH_2)_p$ - $R_3$ dans laquelle :

p est un nombre entier de 0 à 3 ;
$R_3$ représente un groupe CN, un groupe OH, un groupe CCH, un groupe alcoxy-carbonyle en $C_1$ à $C_3$ ou un groupe alkylé en $C_1$ à $C_3$ de cyanométhyle ;

2) une réaction d'acylation du produit issu de la réaction 1) par un réactif tel que l'isocyanate de phényle.

**11.** Procédé de préparation d'un composé de formule particulière (I-e) selon la Revendication 8 ou la Revendication 9, caractérisé en ce qu'il comprend :

1) une réaction de condensation entre un aldéhyde aromatique de formule :

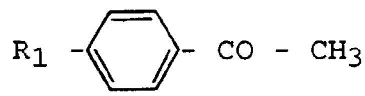

dans laquelle :

$R_1$ représente un groupe benzyloxy ou phényléthoxy ; et :

* soit une hydrazine de formule : $NH_2$- NH - $(CH_2)_p$ - $R_3$ dans laquelle :

p est un nombre entier de 0 à 3 ;
$R_3$ représente un groupe CN, un groupe OH, un groupe CCH, un groupe carboxy-carbonyle en $C_1$ à $C_3$ ou un groupe alkylé en $C_1$ à $C_3$ de cyanométhyle ;

* soit l'hydrazine de formule : $NH_2$- NH - CO - $CH_3$ ; 2) une réaction :
* soit d'acylation du produit issu de la réaction 1) par un réactif tel que le chlorure d'acide ;
* soit d'alkylation du produit issu de la réaction 1) par un réactif de formule : Hal - $(CH_2)_p$ - C ≡ Z

dans laquelle :

p est un nombre entier de 0 à 3 ;
Hal représente un atome d'halogène ;
Z représente un groupe CH ou un atome d'azote,

ou par un réactif de formule :

$$Br - (CH_2)_p - COO - \text{alkyle en } C_1 \text{ à } C_3$$

dans laquelle p est un nombre entier de 0 à 3.

## Patentansprüche

**1.** Verwendung einer Verbindung der Formel (I) :

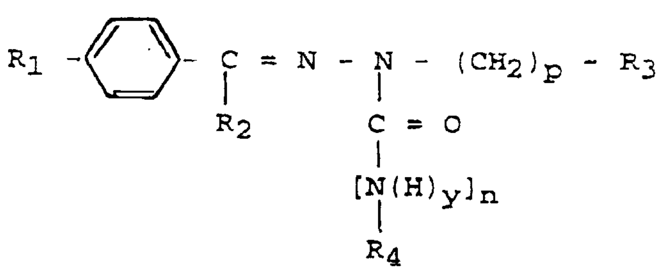

(I)

in der:

$R_1$ ein Wasserstoffatom, eine Phenylethoxygruppe oder eine Benzyloxygruppe, in der der Phenylkern gegebenenfalls mit einem oder mehreren Halogenatomen und/oder mehreren $NO_2$-Gruppen und/oder einer oder mehreren $C_1$-$C_3$-Alkylgruppen und/oder einer oder mehreren $C_1$-$C_3$-Alkoxygruppen substituiert ist, bedeutet;
p eine ganze Zahl von 0 bis 3 ist;
$R_3$ ein Wasserstoffatom, eine CN-Gruppe, eine Hydroxylgruppe, eine CCH-Gruppe, eine $C_1$-$C_3$-Alkoxycarbonylgruppe oder eine $C_1$-$C_3$-alkylierte Cyanomethylgruppe bedeutet;
$R_2$ ein Wasserstoffatom oder eine $C_1$-$C_3$-Alkylgruppe bedeutet;
n 0 oder 1 ist, mit der Maßgabe, daß

- wenn n = 0, $R_4$ eine $C_1$-$C_3$-Alkylgruppe oder eine $C_1$-$C_3$-Alkoxygruppe bedeutet, wohingegen
- wenn n = 1, y = 1 und $R_4$ eine $C_1$-$C_3$-Alkylgruppe, eine $C_1$-$C_3$-Alkoxygruppe oder eine Phenylgruppe bedeutet, zur Herstellung eines Medikaments, das eine inhibitorische Aktivität auf die Monoaminoxidase B besitzt.

**2.** Verwendung einer Verbindung nach Anspruch 1, dadurch **gekennzeichnet**, daß $R_1$ eine Benzyloxygruppe bedeutet, in der der Phenylkern gegebenenfalls mit einem oder mehreren Halogenatomen und/oder mit einer oder mehreren $NO_2$-Gruppen und/oder mit einer oder mehreren $C_1$-$C_3$-Alkylgruppen und/oder mit einer oder mehreren $C_1$-$C_3$-Alkoxygruppen substituiert ist.

**3.** Verwendung einer Verbindung nach Anspruch 1 oder Anspruch 2, dadurch **gekennzeichnet**, daß die Verbindung ausgewählt ist aus den folgenden Verbindungen:

. 4-(Benzyloxy)benzaldehydacetyl-(2-cyanoethyl)hydrazon (I-a2),
. 4-[(4-Methylbenzyl)oxy]benzaldehydacetyl-(2-cyanoethyl)hydrazon (I-a2.1),
. 4-[(4-Nitrobenzyl)oxy]benzaldehydacetyl-(2-cyanoethyl)hydrazon (I-a2.2),
. 4-[(4-Chlorbenzyl)oxy]benzaldehydacetyl-(2-cyanoethyl)hydrazon (I-a2.3),
. 4-[(4-Methoxybenzyl)oxy]benzaldehydacetyl-(2-cyanoethyl)hydrazon (I-a2.4),
. 4- [(2,4-Dichlorbenzyl)oxy]benzaldehydacetyl-(2-cyanoethyl)hydrazon (I-a2.5),
. 4-[(2-Chlorobenzyl)oxy]benzaldehydacetyl-(2-cyanoethyl)hydrazon (I-a2.6),
. 4-(Benzyloxy)benzaldehydacetyl-(2-hydroxyethyl)hydrazon (I-a4),
. 4-(Benzyloxy)benzaldehydacetyl-(2-methoxycarbonylethyl)hydrazon (I-a5),
. 4-(2-Phenylethoxy)benzaldehydacetyl-(2-cyanoethyl)hydrazon (I-a6),
. 4-(Benzyloxy)benzaldehydacetyl-(2-cyanopropyl)hydrazon (I-a7),
. 4- (Benzyloxy)benzaldehydacetyl(cyanomethyl)hydrazon(I-a8),
. 4-(Benzyloxy)benzaldehydacetyl-(3-cyanopropyl)hydrazon (I-a9),
. 4-(Benzyloxy)benzaldehydacetylpropargylhydrazon (1-a10),
. 4-(Benzyloxy)benzaldehyd-(2-cyanoethyl)-(ethoxycarbonyl)hydrazon (I-b2),
. 4-(Benzyloxy)benzaldehyd-(2-hydroxyethyl)-(ethoxycarbonyl)hydrazon (I-b4),
. Benzaldehyd-(2-cyanoethyl)-(N-methylcarbamoyl)hydrazon (I-c1),
. 4-(Benzyloxy)benzaldehyd-(2-cyanoethyl)-(N-methylcarbamoyl)hydrazon) (I-c2),
. Benzaldehyd-(2-cyanoethyl)-(N-phenylcarbamoyl)hydrazon (I-d1),
. 4-(Benzyloxy)benzaldehyd-(2-cyanoethyl)-(N-phenylcarbamoyl)hydrazon (I-d2), und
. 4-(benzyloxy)acetophenonacetyl-(2-cyanoethyl)hydrazon (I-e1).

**4.** Medikament, dadurch **gekennzeichnet**, daß es wenigstens einen Wirkstoff mit einer der folgenden speziellen Formeln enthält:

(I-a)

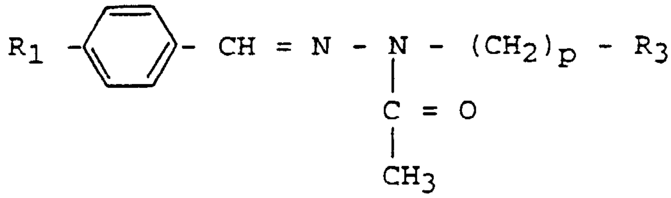

(I-b) 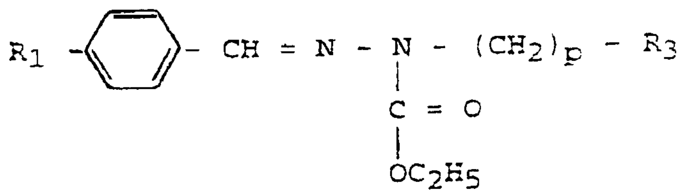

(I-c) 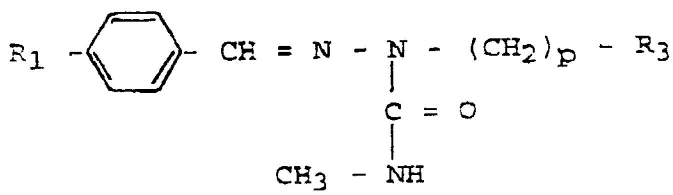

(I-d) 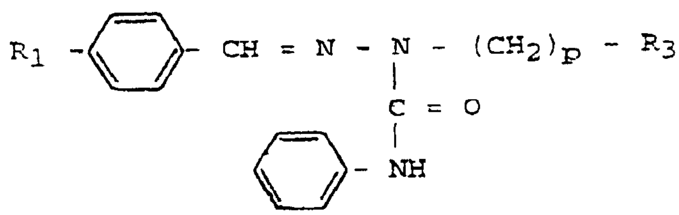

(I-e) 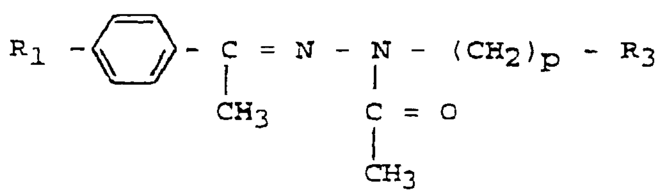

in denen:

$R_1$ ein Wasserstoffatom, eine Phenylethoxygruppe oder eine Benzyloxygruppe, in der der Phenylkern gegebenenfalls mit einem oder mehreren Halogenatomen und/oder einer oder mehreren $NO_2$-Gruppen und/oder einer oder mehreren $C_1$-$C_3$-Alkylgruppen und/oder einer oder mehreren $C_1$-$C_3$-Alkoxygruppen substituiert ist, bedeutet, mit der Maßgabe, daß $R_1$ in der speziellen Formel (I-e) nicht Wasserstoff bedeutet;
p eine ganze Zahl von 0 bis 3 ist;
$R_3$ eine CN-Gruppe, eine OH-Gruppe, eine CCH-Gruppe, eine $C_1$-$C_3$-Alkoxycarbonylgruppe oder eine $C_1$-$C_3$-alkylierte Cyanomethylgruppe bedeutet.

**5.** Medikament nach Anspruch 4, dadurch **gekennzeichnet**, daß $R_1$ eine Benzyloxygruppe bedeutet, in der der Phenylkern gegebenenfalls mit einem oder mehreren Halogenatomen und/oder einer oder mehreren $NO_2$-Gruppen und/oder einer oder mehreren $C_1$-$C_3$-Alkylgruppen und/oder einer oder mehreren $C_1$-$C_3$-Alkoxygruppen substituiert ist.

**6.** Medikament nach Anspruch 4, dadurch **gekennzeichnet**, daß der Wirkstoff aus den folgenden Verbindungen ausgewählt ist:

. 4-(Benzyloxy)benzaldehydacetyl-(2-cyanoethyl)hydrazon (I-a2),

- 4-[(4-Methylbenzyl)oxy]benzaldehydacetyl-(2-cyanoethyl)hydrazon (I-a2.1),
- 4-[(4-Nitrobenzyl)oxy]benzaldehydacetyl-(2-cyanoethyl)hydrazon (I-a2.2),
- 4-[(4-Chlorbenzyl)oxy]benzaldehydacetyl-(2-cyanoethyl)hydrazon (I-a2.3),
- 4-[(4-Methoxybenzyl)oxy]benzaldehydacetyl-(2-cyanoethyl)-hydrazon (I-a2.4),
- 4-[(2,4-Dichlorbenzyl)oxy]benzaldehydacetyl-(2-cyanoethyl)hydrazon (I-a2.5),
- 4-[(2-Chlorbenzyl)oxy]benzaldehydacetyl-(2-cyanoethyl)hydrazon (I-a2.6),
- 4-(Benzyloxy)benzaldehydacetyl-(2-hydroxyethyl)hydrazon (I-a4),
- 4-(Benzyloxy)benzaldehydacetyl-(2-methoxycarbonylethyl)hydrazon (I-a5),
- 4- (2-Phenylethoxy)benzaldehydacetyl-(2-cyanoethyl)hydrazon (I-a6),
- 4-(Benzyloxy)benzaldehydacetyl-(2-cyanopropyl)hydrazon (I-a7),
- 4-(Benzyloxy)benzaldehydacetyl-(cyanomethyl)hydrazon (I-a8),
- 4-(Benzyloxy)benzaldehydacetyl-(3-cyanopropyl)hydrazon (I-a9),
- 4-(Benzyloxy)benzaldehydacetyl-propargylhydrazon (1-a10),
- 4-(Benzyloxy)benzaldehyd-(2-cyanoethyl)-(ethoxycarbonyl)hydrazon (I-b2),
- 4-(Benzyloxy)benzaldehyd-(2-hydroxyethyl)-(ethoxycarbonyl)hydrazon (I-b4),
- Benzaldehyd-(2-cyanoethyl)-(N-methylcarbamoyl)hydrazon (I-c1),
- 4-(Benzyloxy)benzaldehyd-(2-cyanoethyl)-(N-methylcarbamoyl)hydrazon (I-c2),
- Benzaldehyd-(2-cyanoethyl)-(N-phenylcarbamoyl)hydrazon (I-d1),
- 4-(Benzyloxy)benzaldehyd-(2-cyanoethyl)-(N-phenylcarbamoyl)hydrazon (I-d2), und
- 4-(benzyloxy)acetophenonacetyl-(2-cyanoethyl)hydrazon (I-e1).

**7.** Medikament nach Anspruch 6, dadurch **gekennzeichnet**, daß der Wirkstoff 4-(Benzyloxybenzaldehydacetyl-(2-cyanoethyl)hydrazon (I(a2) ist.

**8.** Verbindung, dadurch **gekennzeichnet**, daß sie eine der folgenden speziellen Formel aufweist:

(I-d)

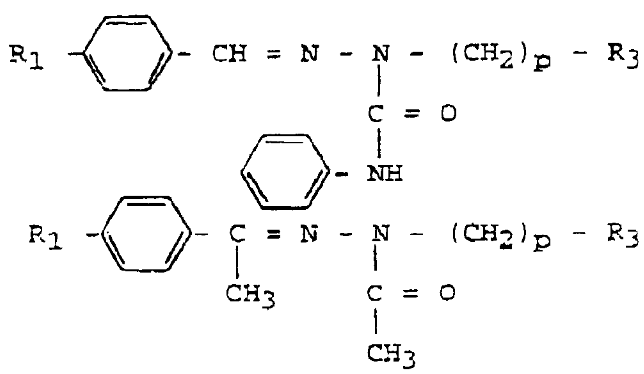

(I-e)

in denen:

$R_1$ ein Wasserstoffatom, eine Benzyloxygruppe oder eine Phenylethoxygruppe bedeutet, mit der Maßgabe, daß $R_1$ nicht Wasserstoff in der speziellen Formel (I-e) bedeutet;
p eine ganze Zahl von 0 bis 3 ist;
$R_3$ eine CN-Gruppe, eine OH-Gruppe, eine CCH-Gruppe, eine $C_1$-$C_3$-Alkoxycarbonylgruppe oder eine $C_1$-$C_3$-alkylierte Cyanomethylgruppe bedeutet.

**9.** Verbindung nach Anspruch 8, dadurch **gekennzeichnet**, daß sie aus den folgenden Verbindungen ausgewählt ist:

- Benzaldehyd-(2-cyanoethyl)-(N-phenylcarbamoyl)hydrazon (I-d1),
- 4-(Benzyloxy)benzaldehyd-(2-cyanoethyl)-(N-phenylcarbamoyl)hydrazon (I-d2), und
- 4-(Benzyloxy)acetophenonacetyl-(2-cyanoethyl)hydrazon (I-e1).

**10.** Verfahren zur Herstellung einer Verbindung der speziellen Formel (I-d) nach Anspruch 8 oder Anspruch 9, dadurch **gekennzeichnet**, daß es die folgenden Stufen umfaßt:

1) eine Kondensationsreaktion zwischen einem aromatischen Aldehyd der Formel:

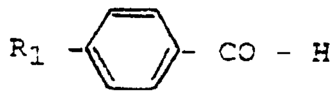

in der

$R_1$ ein Wasserstoffatom, eine Benzyloxy- oder Phenylethoxygruppe bedeutet;
und einem Hydrazin der Formel: $NH_2$-NH-$(CH_2)_p$-$R_3$,
in der
p eine ganze Zahl von 0 bis 3 ist;
$R_3$ eine CN-Gruppe, eine OH-Gruppe, eine CCH-Gruppe, eine $C_1$-$C_3$-Alkoxycarbonylgruppe oder eine $C_1$-$C_3$-alkylierte Cyanomethylgruppe bedeutet;

2) eine Acylierungsreaktion des in der Stufe 1) erhaltenen Produkts mit einem Reagens wie Phenylisocyanat.

**11.** Verfahren zur Herstellung einer Verbindung der speziellen Formel (I-e) nach Anspruch 8 oder Anspruch 9, dadurch **gekennzeichnet**, daß es die folgenden Stufen umfaßt:

1) eine Kondensationsreaktion zwischen einem aromatischen Aldehyd der Formel:

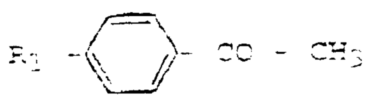

in der

$R_1$ eine Benzyloxy- oder Phenylethoxygruppe bedeutet; und:

* entweder einem Hydrazin der Formel: $NH_2$-NH-$(CH_2)_p$-$R_3$, in der:

p eine ganze Zahl von 0 bis 3 ist;
$R_3$ eine CN-Gruppe, eine OH-Gruppe, eine CCH-Gruppe, eine $C_1$-$C_3$-Carboxycarbonylgruppe oder eine $C_1$-$C_3$-alkylierte Cyanomethylgruppe bedeutet;

* oder einem Hydrazin der Formel: $NH_2$-NH-CO-$CH_3$;

2) eine Reaktion, bei der es sich

* entweder um eine Acylierung des in der Stufe 1) gewonnen Produkts mit einem Reagens wie Salzsäure;
* oder um eine Alkylierung des in der Stufe 1) erhaltenen Produktes mit einem Reagens der Formel: Hal-$(CH_2)$p-C≡Z, in der:

p eine ganze Zahl von 0 bis 3 ist;
Hal ein Halogenatom bedeutet;
Z eine CH-Gruppe oder ein Stickstoffatom bedeutet,

oder mit einem Reagens der Formel:

$$Br\text{-}(CH_2)p\text{-}COO\text{-}(C_1\text{-}C_3)\text{-}Alkyl$$

und in der p eine ganze Zahl von 0 bis 3 ist, handelt.

## Claims

1. Use of a compound of formula (I) :

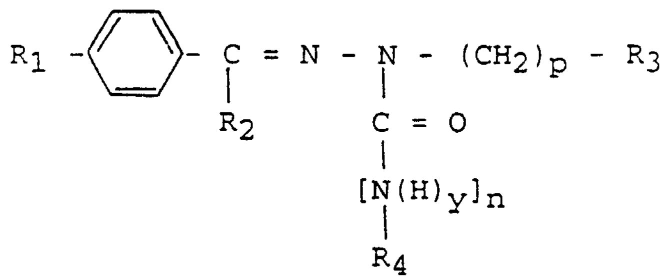

(I)

wherein:

$R_1$ denotes a hydrogen atom, a phenylethoxy group or a benzyloxy group wherein the phenyl nucleus is optionally substituted by one or more halogen atoms and/or a plurality of $NO_2$ groups and/or one or more $C_{1-3}$-alkyl groups and/or one or more $C_{1-3}$-alkoxy groups;

p is an integer from 0 to 3;

$R_3$ denotes a hydrogen atom, a CN group, a hydroxyl group, a CCH group, a $C_{1-3}$-alkoxycarbonyl group or a $C_{1-3}$-alkylated cyanomethyl group;

$R_2$ denotes a hydrogen atom or a $C_{1-3}$-alkyl group;

n is equal to 0 or 1, in which case

- if n = 0, $R_4$ denotes a $C_{1-3}$-alkyl group or a $C_{1-3}$-alkoxy group, whereas
- if n = 1, y = 1 and $R_4$ denotes a $C_{1-3}$-alkyl group, a $C_{1-3}$-alkoxy group or a phenyl group;

for the preparation of a medicament having an inhibitory effect on Monoamine Oxidase B.

2. Use of a compound according to claim 1, characterised in that $R_1$ denotes a benzyloxy group wherein the phenyl nucleus is optionally substituted by one or more halogen atoms and/or one or more $NO_2$ groups and/or one or more $C_{1-3}$-alkyl groups and/or one or more $C_{1-3}$-alkoxy groups.

3. Use of a compound according to claim 1 or 2, characterised in that the compound is selected from among the following compounds:

4-(benzyloxy)benzaldehyde-acetyl (2-cyanoethyl)hydrazone (I-a2),
4-[(4-methylbenzyl)oxy]benzaldehyde-acetyl(2-cyanoethyl)-hydrazone (I-a2.1),
4-[(4-nitrobenzyl)oxy]benzaldehyde-acetyl(2-cyanoethyl)-hydrazone (I-a2.2),
4-[(4-chlorobenzyl)oxy]benzaldehyde-acetyl(2-cyanoethyl)-hydrazone (I-a2.3),
4-[(4-methoxybenzyl)oxy]benzaldehyde-acetyl(2-cyanoethyl)-hydrazone (I-a2.4),
4-[(2,4-dichlorobenzyl)oxy]benzaldehyde-acetyl(2-cyanoethyl)-hydrazone (I-a2.5),
4-[(2-chlorobenzyl)oxy]benzaldehyde-acetyl(2-cyanoethyl)-hydrazone (I-a2.6),
4-(benzyloxy)benzaldehyde-acetyl (2-hydroxyethyl)hydrazone (I-a4),
4-(benzyloxy)benzaldehyde-acetyl (2-methoxycarbonylethyl)-hydrazone (I-a5),
4-(2-phenylethoxy)benzaldehyde-acetyl (2-cyanoethyl)-hydrazone (I-a6),
4-(benzyloxy)benzaldehyde-acetyl (2-cyanopropyl)hydrazone (I-a7),
4-(benzyloxy)benzaldehyde-acetyl (cyanomethyl)hydrazone (I-a8),
4-(benzyloxybenzaldehyde-acetyl (3-cyanopropyl)hydrazone (I-a9),
4-(benzyloxy)benzaldehyde-acetylpropargyl-hydrazone (I-a10),
4-(benzyloxy)benzaldehyde-(2-cyanoethyl)(ethoxycarbonyl)-hydrazone (I-b2),
4-(benzyloxy)benzaldehyde-(2-hydroxyethyl)(ethoxycarbonyl)-hydrazone (I-b4),

benzaldehyde-(2-cyanoethyl)-(N-methylcarbamoyl)hydrazone (I-c1),
4-(benzyloxy)benzaldehyde-(2-cyanoethyl)-(N-methylcarbamoyl)-hydrazone (I-c2),
benzaldehyde-(2-cyanoethyl)-(N-phenylcarbamoyl)hydrazone (I-d1),
4-(benzyloxy)benzaldehyde-(2-cyanoethyl)-(N-phenylcarbamoyl)-hydrazone (I-d2), and
4-(benzyloxy)acetophenone-acetyl- (2-cyanoethyl)hydrazone (I-e1).

**4.** Medicament, characterised in that it comprises at least one active substance which corresponds to one of the following individual formulae

(I-a)

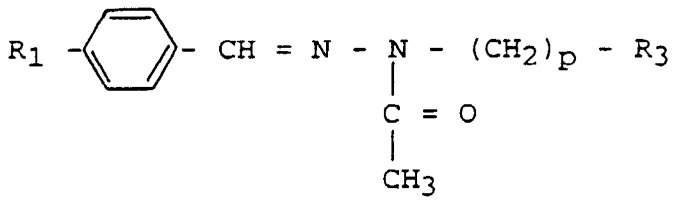

(I-b)

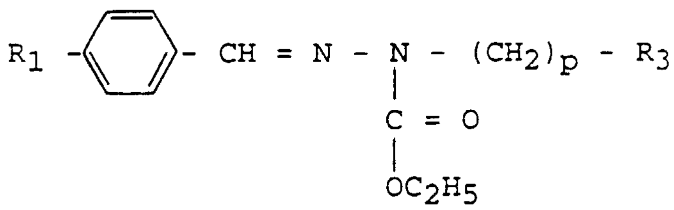

(I-c)

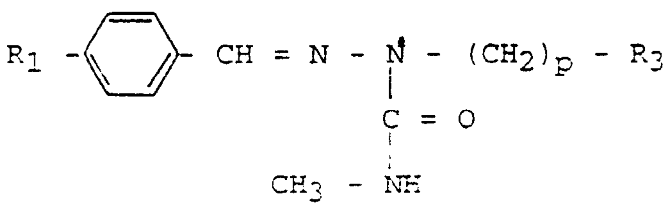

(I-d)

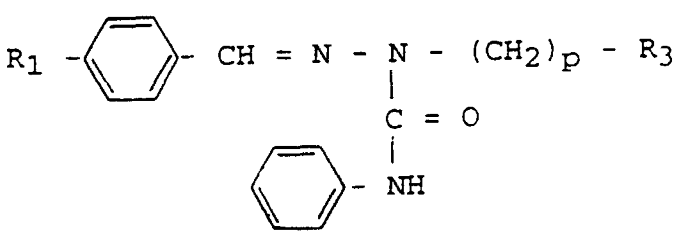

(I-e)

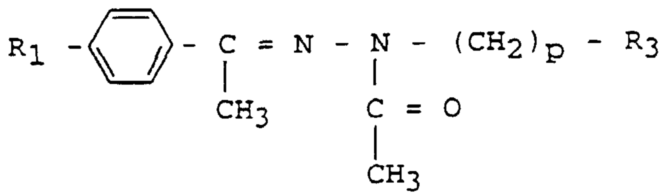

wherein:

$R_1$ denotes a hydrogen atom, a phenylethoxy group or a benzyloxy group wherein the phenyl nucleus is optionally substituted by one or more halogen atoms and/or one or more $NO_2$ groups and/or one or more $C_{1-3}$-alkyl groups and/or one or more $C_{1-3}$-alkoxy groups, with the proviso that $R_1$ is different from a hydrogen atom in the individual formula (I-e);

p is an integer from 0 to 3;

$R_3$ denotes a CN group, an OH group, a CCH group, a $C_{1-3}$-alkoxycarbonyl group or a $C_{1-3}$-alkylated cyanomethyl group.

**5.** Medicament according to claim 4, characterised in that $R_1$ denotes a benzyloxy group wherein the phenyl nucleus is optionally substituted by one or more halogen atoms and/or one or more $NO_2$ groups and/or one or more $C_{1-3}$-alkyl groups and/or one or more $C_{1-3}$-alkoxy groups.

**6.** Medicament according to claim 4, characterised in that the active substance is selected from among the following compounds:

4-(benzyloxy)benzaldehyde-acetyl (2-cyanoethyl)hydrazone (I-a2),
4-[(4-methylbenzyl)oxy]benzaldehyde-acetyl(2-cyanoethyl)-hydrazone (I-a2.1),
4-[(4-nitrobenzyl)oxy]benzaldehyde-acetyl(2-cyanoethyl)-hydrazone (I-a2.2),
4-[(4-chlorobenzyl)oxy]benzaldehyde-acetyl(2-cyanoethyl)-hydrazone (I-a2.3),
4-[(4-methoxybenzyl)oxy]benzaldehyde-acetyl(2-cyanoethyl)-hydrazone (I-a2.4),
4-[(2,4-dichlorobenzyl)oxy]benzaldehyde-acetyl(2-cyanoethyl)-hydrazone (I-a2.5),
4-[(2-chlorobenzyl)oxy]benzaldehyde-acetyl(2-cyanoethyl)-hydrazone (I-a2.6),
4-(benzyloxy)benzaldehyde-acetyl (2-hydroxyethyl)-hydrazone (I-a4),
4-(benzyloxy)benzaldehyde-acetyl (2-methoxycarbonylethyl)-hydrazone (I-a5),
4-(2-phenylethoxy)benzaldehyde-acetyl (2-cyanoethyl)-hydrazone (I-a6),
4-(benzyloxy)benzaldehyde-acetyl (2-cyanopropyl)-hydrazone (I-a7),
4-(benzyloxy)benzaldehyde-acetyl (cyanomethyl)-hydrazone (I-a8),
4-(benzyloxy)benzaldehyde-acetyl (3-cyanopropyl)-hydrazone (I-a9),
4-(benzyloxy)benzaldehyde-acetylpropargyl-hydrazone (I-a10),
4-(benzyloxy)benzaldehyde-(2-cyanoethyl)(ethoxycarbonyl)-hydrazone (I-b2),
4-(benzyloxy)benzaldehyde-(2-hydroxyethyl)-(ethoxycarbonyl)-hydrazone (I-b4),
benzaldehyde-(2-cyanoethyl)-(N-methylcarbamoyl)-hydrazone (I-c1),
4-(benzyloxy)benzaldehyde-(2-cyanoethyl)-(N-methylcarbamoyl)-hydrazone (I-c2),
benzaldehyde-(2-cyanoethyl)-(N-phenylcarbamoyl)-hydrazone (I-d1),
4-(benzyloxy)benzaldehyde-(2-cyanoethyl)-(N-phenylcarbamoyl)-hydrazone (I-d2), and
4-(benzyloxy)acetophenone-acetyl-(2-cyanoethyl)-hydrazone (I-e1).

**7.** Medicament according to claim 6, characterised in that the active substance is 4-(benzyloxy)benzaldehydeacetyl (2-cyanoethyl)-hydrazone (I-a2).

**8.** Compound, characterised in that it corresponds to one of the individual formulae:

(I-d)

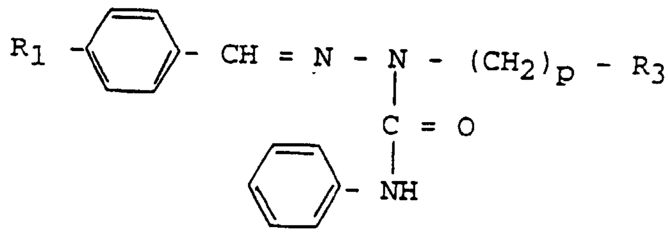

(I-e)

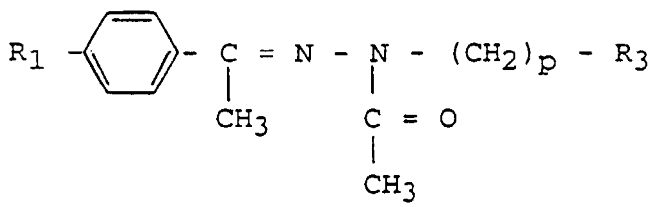

wherein:

$R_1$ denotes a hydrogen atom, a benzyloxy group or a phenylethoxy group, with the proviso that $R_1$ is different from a hydrogen atom in the individual formula (I-e);

p is an integer from 0 to 3;

$R_3$ denotes a CN group, an OH group, a CCH group, a $C_{1-3}$-alkoxycarbonyl group or a $C_{1-3}$-alkylated cyanomethyl group.

**9.** Compound according to claim 8, characterised in that it is selected from among the following compounds:

benzaldehyde-(2-cyanoethyl)-(N-phenylcarbamoyl)hydrazone (I-d1),
4-(benzyloxy)benzaldehyde-(2-cyanoethyl)-(N-phenylcarbamoyl)-hydrazone (I-d2), and
4-(benzyloxy)acetophenone-acetyl-(2-cyanoethyl)hydrazone (I-e1).

**10.** Process for preparing a compound of the individual formula (I-d) according to claim 8 or 9, characterised in that it comprises:

1) a condensation reaction between an aromatic aldehyde of formula:

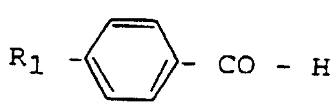

wherein:

$R_1$ denotes a hydrogen atom or a benzyloxy or phenylethoxy group;
and a hydrazine of formula: $NH_2$- NH - $(CH_2)_p$ - $R_3$ wherein:
p is an integer from 0 to 3 and
$R_3$ denotes a CN group, an OH group, a CCH group, a $C_{1-3}$-alkoxycarbonyl group or a $C_{1-3}$-alkylated cyanomethyl group;

2) an acylation reaction of the product obtained in reaction 1, with a reagent such as phenyl isocyanate.

**11.** Process for preparing a compound of individual formula (I-e) according to claim 8 or claim 9, characterised in that

it comprises:

1) a condensation reaction between an aromatic aldehyde of formula:

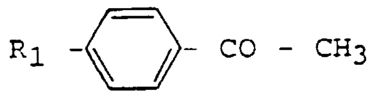

wherein:

$R_1$ denotes a benzyloxy or phenylethoxy group;
and:

* either a hydrazine of formula: $NH_2 - NH - (CH_2)_p - R_3$ wherein:

p is an integer from 0 to 3 and
$R_3$ denotes a CN group, an OH group, a CCH group, a $C_{1-3}$-carboxycarbonyl group or a $C_{1-3}$-alkylated cyanomethyl group;

* or a hydrazine of formula: $NH_2 - NH - CO - CH_3$ ;

2) a reaction:

* either of acylation of the product obtained in reaction 1) with a reagent such as the acid chloride ;
* or of alkylation of the product obtained in reaction 1) with a reagent of formula: Hal - $(CH_2)_p - C \equiv Z$ wherein:

p is an integer from 0 to 3;
Hal denotes a halogen atom and
Z denotes a CH group or a nitrogen atom,
or with a reagent of formula:

$$Br - (CH_2)_p - COO - C_{1-3}\text{-alkyl}$$

wherein p is an integer from 0 to 3.

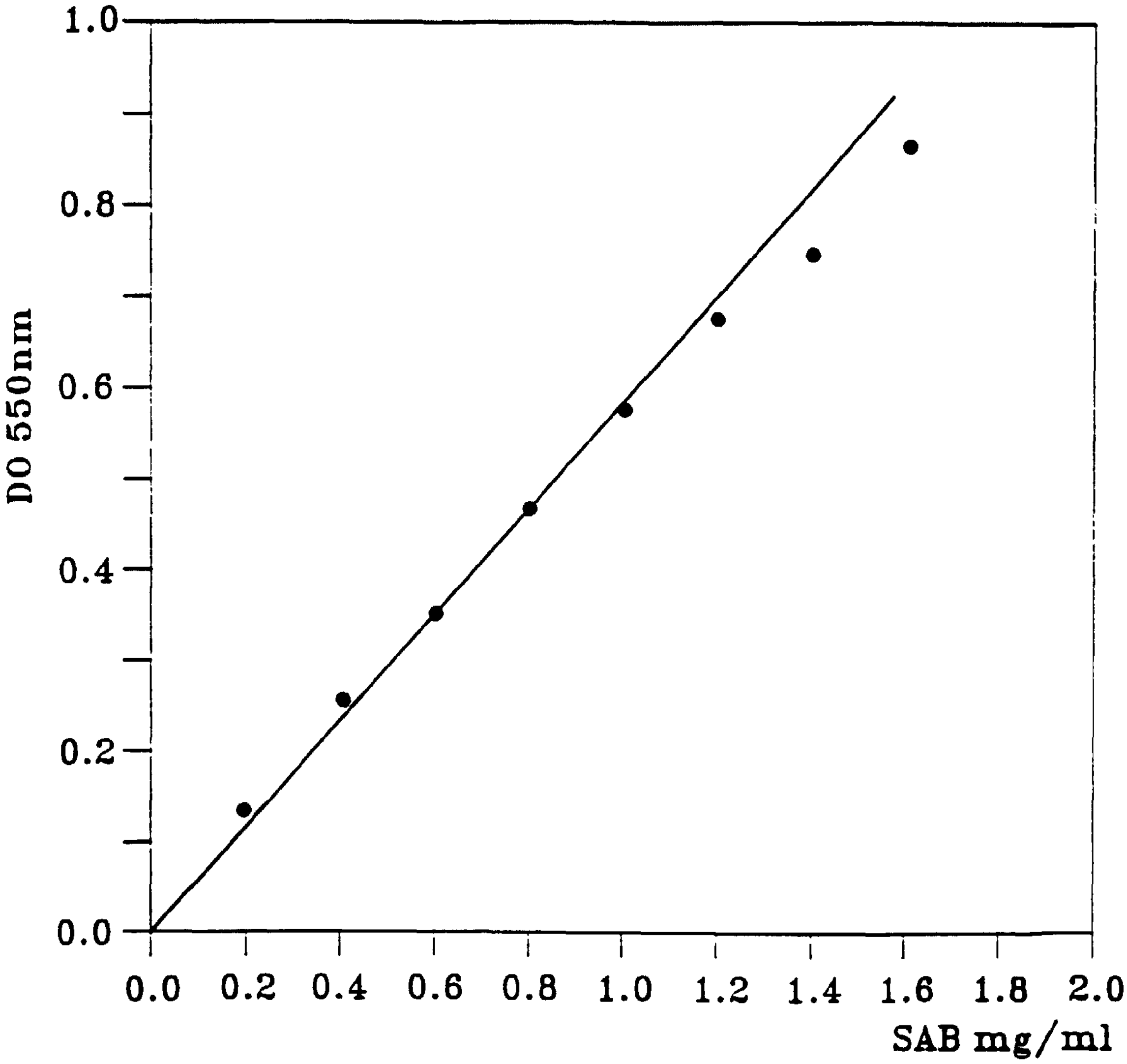


FIG.1

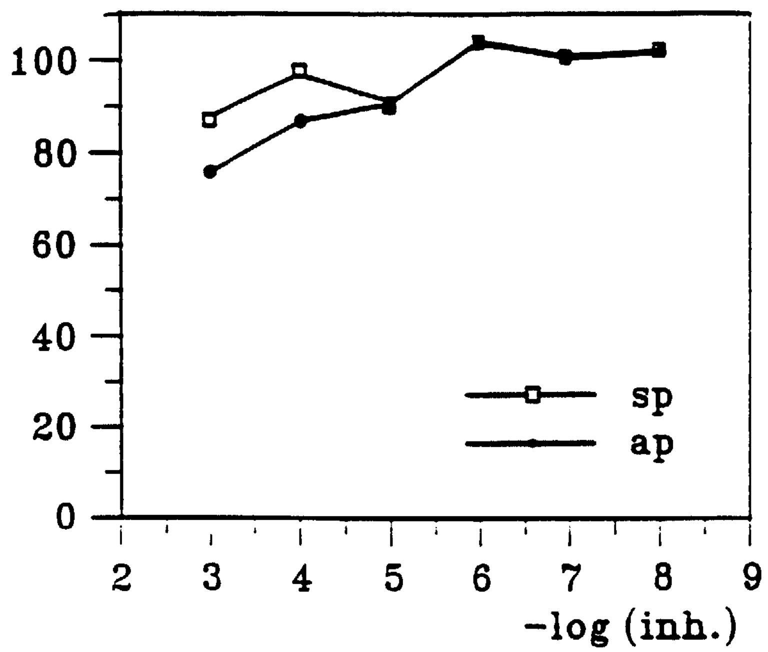


FIG.2A MAO A

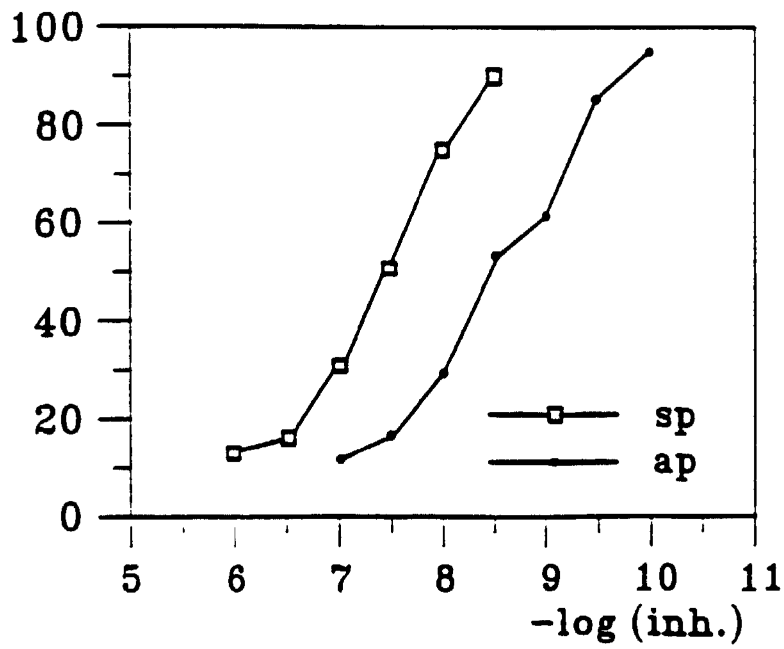


MAO B

FIG.2B

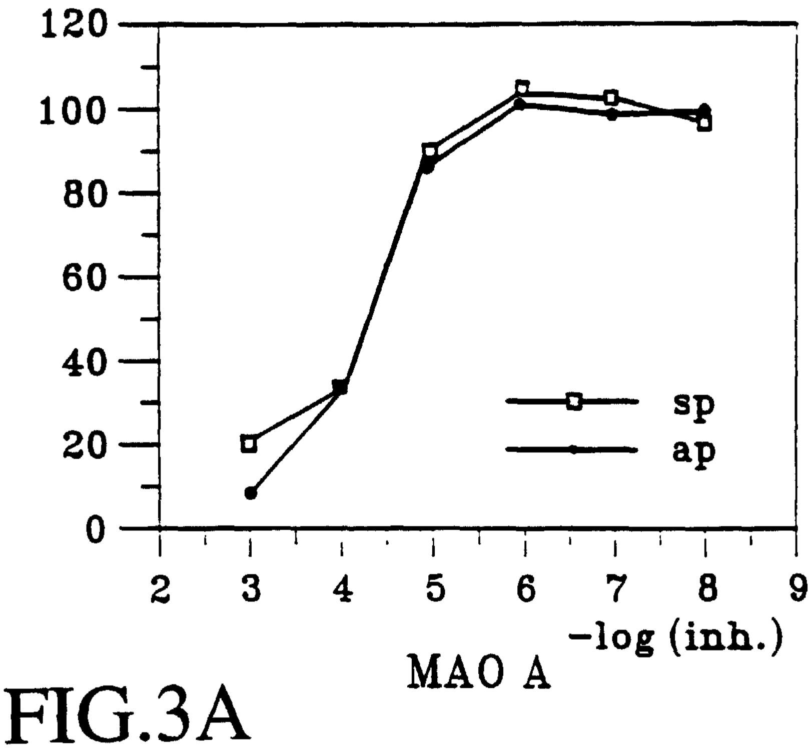
120
100
80
60
40
20
0
2
3
4
5
6
7
8
9
-log (inh.)
MAO A
sp
ap

FIG.3A

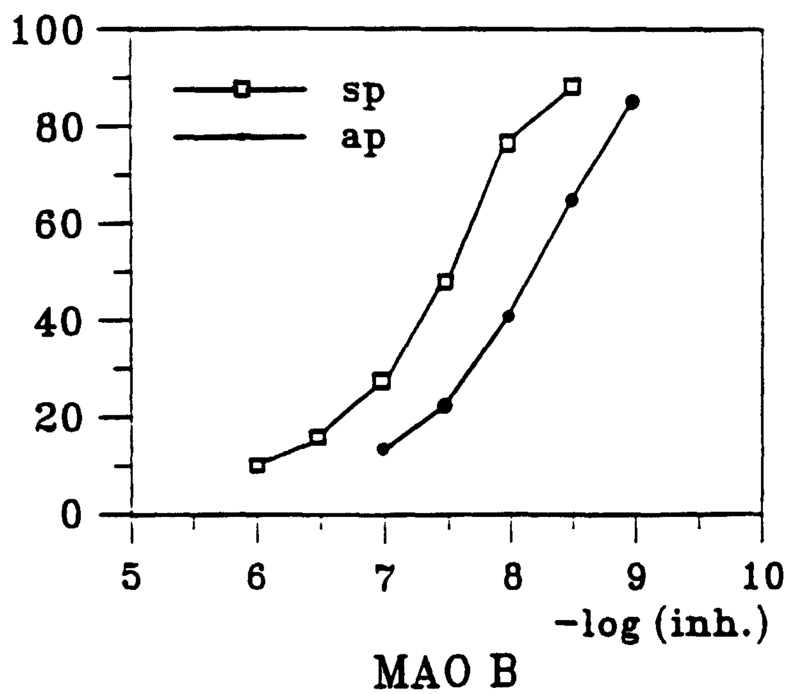
100
80
60
40
20
0
5
6
7
8
9
10
-log (inh.)
MAO B
sp
ap

FIG.3B

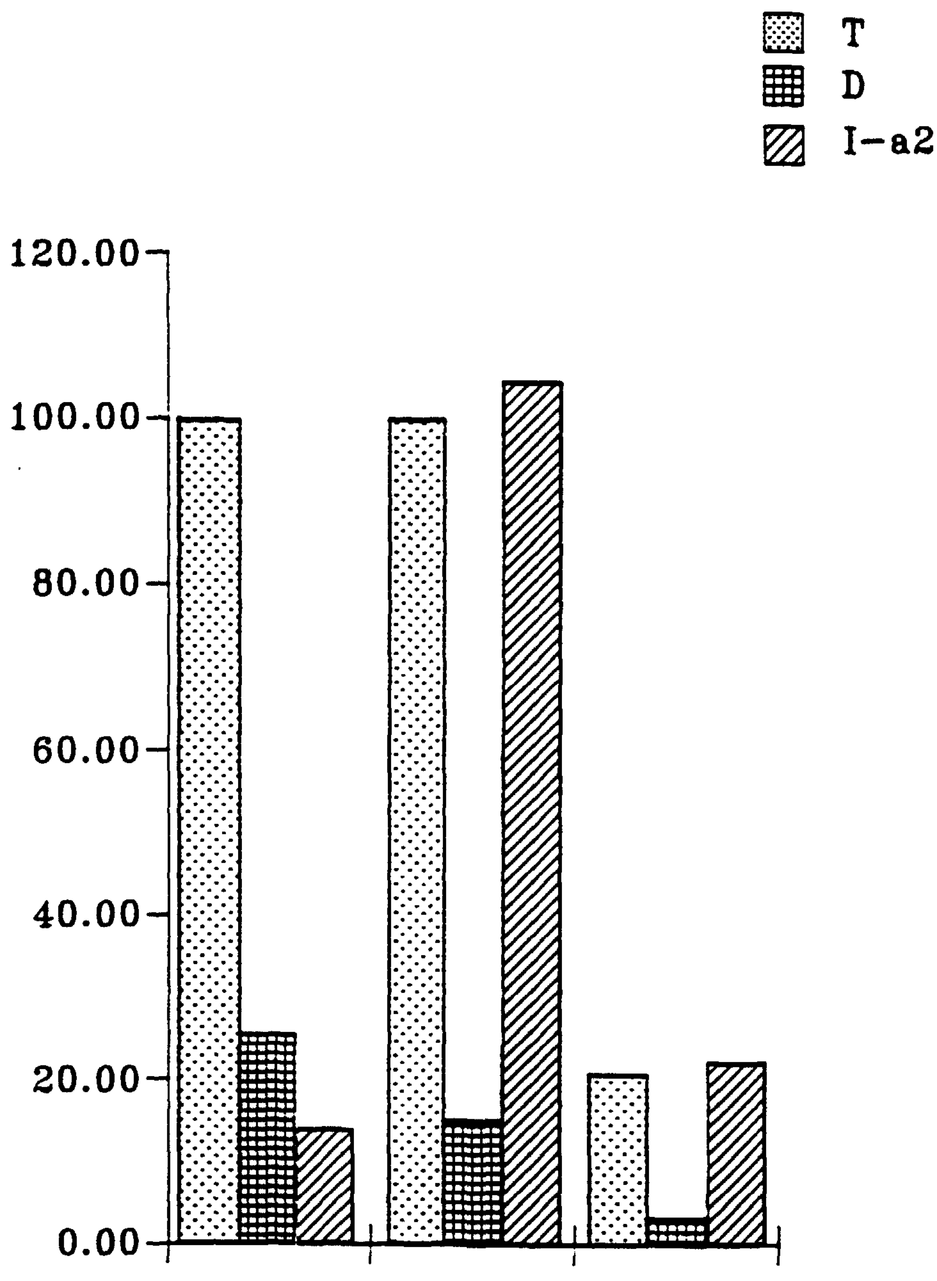


FIG.4